# EUROPEAN PATENT SPECIFICATION

(11) **EP 0 792 271 B1**
(45) Date of publication and mention of the grant of the patent: **27.02.2002**
(21) Application number: 95941407.9
(22) Date of filing: 13.11.1995
(51) Int. Cl.: C07D 405/14, A61K 31/415, C07D 307/54, A61K 31/34

(54) **FURAN DERIVATIVES FOR INHIBITING PNEUMOCYSTIS CARINII PNEUMONIA, GIARDIA LAMBLIA AND CRYPTOSPORIDIUM PARVUM**
FURANDERIVATE ZUR HEMMUNG VON PNEUMoCYSTIS CARINII PNEUMONIA, GIARDIA LAMBLIA UND CRYPTOSPORIDIUM PARVUM
DERIVES DE FURANNE POUR INHIBER LA PNEUMONIE A PNEUMOCYSTIS CARINII, LA GIARDIA LAMBLIA ET LE CRYPTOSPORIDUM PARVUM

(30) Priority: 14.11.1994 US 339487
(43) Date of publication of application: 03.09.1997
(73) Proprietor: GEORGIA STATE UNIVERSITY RESEARCH FOUNDATION, INC., Atlanta, GA 30303-3083 (US); THE UNIVERSITY OF NORTH CAROLINA AT CHAPEL HILL, Chapel Hill, North Carolina 27599-4100 (US); AUBURN UNIVERSITY, Auburn , AL 36849-5112 (US)
(72) Inventor: BOYKIN, David, W., Atlanta, GA 30306 (US); DYKSTRA, Christine, C., Auburn, AL 36830 (US); TIDWELL, Richard R., Route 3 Box 212K, Pittsboro, NC 27312 (US); HALL, James, E., Chapel Hill, NC 27514 (US); WILSON, W., David, Atlanta, GA 30307 (US); KUMAR, Arvind, Atlanta, GA 30329 (US); BLAGBURN, Byron, L., Auburn, AL 36830 (US)
(74) Representative: Horner, Martin Grenville
(86) International application number: US9514893
(87) International publication number: WO9615126

(56) References cited:
- J.MED.CHEM., vol. 38, no. 6, 1995 pages 912-916, BOYKIN ET AL 'Dicationic Diarylfurans as Anti-Pneumocystis carinii Agents'
- EXPERIMENTAL PARASITOLOGY, vol. 53, no. 1, 1982 pages 133-144, STECK ET AL 'Trypanosoma rhodesiense: Evaluation of the Antitrypanosomal Action of 2,5-bis (4-Guanylphenyl)furan Dihydrochloride'
- J.MED.CHEM., vol. 20, no. 4, 1977 pages 531-536, DAS ET AL 'Synthesis and Antiprotozoal Activity of 2,5-Bis (guanylphenyl) furans'

## Description

The present invention was made with Government support under Grant Number UO1-A1-3363 from the National Institutes of Health. The Government has certain rights to this invention.

### Field of the Invention

The present invention relates to methods of combatting *Pneumocystis carinii* pneumonia with dicationic compounds. Specifically, the present invention relates to methods of combatting *Pneumocystis carinii* pneumonia with bis-aryl furans and novel bis-aryl furans useful therefor.

### Background of the Invention

Pentamidine is used for the treatment of *Pneumocystis carinii* pneumonia, or "PCP". The importance of pentamidine has escalated recently due to the marked increase of patients suffering from PCP. The increase in the afflicted patient population is an unfortunate consequence of the increasing presence of Acquired Immunodeficiency Syndrome ("AIDS"). It is now estimated that approximately 70 percent of AIDS patients contract PCP. Because of the high incidence of PCP in AIDS patients, pentamidine has found utility not only in the treatment of PCP, but also as prophylaxis, in preventing or delaying the initial onset or recurrence of PCP, especially in AIDS patients. Currently, pentamidine is most commonly administered as a therapeutic agent by intravenous infusion and as a prophylactic agent by aerosol dosage.

However, an unfortunate side effect of pentamidine is its toxicity. Some fatalities have been attributed to severe hypotension, hypoglycemia, and cardiac arrhythmias in patients treated with pentamidine. Contrawise, insufficient dosage dosage may result in dissemination of disease beyond the lung, an occurrence of which is associated with a poor prognosis.

Pentamidine is presently in limited use because of cost and toxicity. Therapeutic drug monitoring is not used because of the cost and complexity of the currently available assay techniques which require the extraction of plasma and High Performance.Liquid Chromatography analysis. As a result, the toxicity of pentamidine is a significant concern, which is driving the market toward the development of pentamidine substitutes capable of avoiding or minimizing the undesirable side effects associated with the use of pentamidine.

The anti-protozoa activity of 2,5-bis(4 guanyl phenyl)furans and elated analogues was investigated by Bijan P Das and David W. Boykin (Journal of Medicinal Chemistry, 20, 531 (1977)) and Edgar A. Steck et al (Experimental Parasitology, 53, 133 (1982)). Although a number of compounds were effective against *trypanosoma rhodesiense* in mice as described by Bijan and Boykin, the efficacy of these compounds were not tested against *Pneumocystis carinii* pneumonia, *Giardia lamblia* or *Cryptosporidium parvum*.

Intervening publication Blakin et al (J Med Chem, 1995, 38, 912 - 916) describes the synthesis of a number of dicationic 2,5-diarylfurans. Several of these compounds were found to be effective in inhibiting topoisomerase II solated from *Giardia lamblia* and were effective against *Pneumocystis carinii* in immuno suppressed rat models.

Accordingly, it is an object of the present invention to provide new methods of treating *Pneumocystis carinii* pneumonia.

### Summary of the Invention

As a first aspect, the present invention provides a use of a compound according to Formula (I): 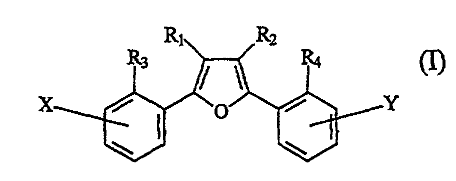 or a pharmaceutically acceptable salt thereof in the manufacture of a medicament for the treatment of *Pneumocystis carinii* pneumonia in a subject in need of such treatment, by administering to said subject an effective *Pneumocystis carinii* pneumonia-combatting amount of the compound according to Formula (I), wherein:
R₁ and R₂ are each independently selected from the group consisting of H, C₁₋₆ alkyl, amino C₁₋₆ alkyl, halogen, and oxy C₁₋₆ alkyl;
R₃ and R₄ are each independently selected from the group consisting of H, C₁₋₆ alkyl, oxy C₁₋₆ alkyl, amino C₁₋₆ alkyl, and halogen; and
X and Y are located in the *para* positions and are 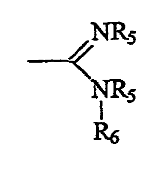 wherein:
   each R₅ is independently selected from the group consisting of H, C₁₋₆ alkyl, C₁₋₆ alkoxy C₁₋₆ alkyl, hydroxy C₁₋₆ alkyl, amino C₁₋₆ alkyl, C₁₋₆ alkylamino C₁₋₆ alkyl, and cyclo C₁₋₆ alkyl, or two R₅ groups together represent C₂-C₁₀ alkyl, hydroxy C₁₋₆ alkyl, or C₁₋₆ alkylene; and
   R₆ is H, hydroxy, C₁₋₆ alkyl, C₁₋₆ alkoxy C₁₋₆ alkyl, hydroxy C₁₋₆ alkyl, amino C₁₋₆ alkyl, C₁₋₆ alkylamino, C₁₋₆ alkylamino C₁₋₆ alkyl, cyclo C₁₋₆ alkyl, hydroxycyclo C₁₋₆ alkyl, or C₁₋₆ alkoxycyclo C₁₋₆ alkyl, in an amount effective to treat *Pneumocystis carinii* pneumonia.

In another embodiment, two R₅ groups together represent 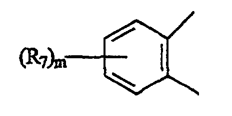 wherein m is from 1-3 and R₇ is H or -CONHR₈NR₉R₁₀, wherein R₈ is C₁₋₆ alkyl, and R₉ and R₁₀ are each independently selected from the group consisting of H and C₁₋₆ alkyl, although these compounds are not currently preferred.

As a second aspect, the present invention provides compounds useful for the treatment of *Pneumocystis carinii* pneumonia. The compounds have the structural Formula (I), described above. In particular, novel compounds useful for the treatment of *Pneumocystis carinii* pneumonia include compounds defined wherein X and Y are located in the *para* position and are each 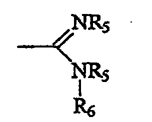 and wherein:
(a) R₁ is H, R₂ is H or C₁₋₆ alkyl, R₃ is H, R₄ is H, R₅ is H, and R₆ is isoalkyl, such as isopropyl, isobutyl, isopentyl, and the like;
(b) R₁ is H, R₂ is H, R₃ is H, R₄ is H, R₅ is H, and R₆ is C₃-C₈ alkoxyalkyl;
(c) R₁ is H, R₂ is H or C₁₋₆ alkyl, R₃ is H, R₄ is H, R₅ is H, and R₆ is alkylhydroxy, such as ethylhydroxy, propylhydroxy, butylhydroxy, pentylhydroxy, and hexylhydroxy;
(d) R₁ is H, R₂ is H or C₁₋₆ alkyl, R₃ is H, R₄ is H, R₅ is H, and R₆ is propoxyethyl;
(e) R₁ is H, R₂ is H or C₁₋₆ alkyl, R₃ is H, R₄ is H, R₅ is H, and R₆ is propoxyisopropyl;
(f) R₁ is H, R₂ is H or loweralkyl, R₃ is H, R₄ is H, R₅ is H, and R₆ is tolyloxy, benzyl, tolyloxy C₁₋₆ alkyl or benzyl C₁₋₆ alkyl; and
(g) R₁ is H, R₂ is H or C₁-C₆ alkyl, R₃ is H, R₄ is H, R₅ is H, and R₆ is C₁-C₆ alkylcyclo C₁-C₆ alkyl; and pharmaceutically acceptable salts thereof.

As a third aspect, the present invention provides use of a compound in the manufacture of a medicament for the treatment of *Giardia lamblia* in a subject in need of such treatment, by administering to said subject an effective *Giardia lamblia -* combatting amount of the compound according to Formula (I). Novel compounds useful for treating *Giardia lamblia* are also disclosed.

As a fourth aspect, the present invention provides use of a compound according to claim 1 in the manufacture of a medicament for the treatment of *Cryptosporidium parvum* in a subject in need of such treatment by administering said subject an effective *Cryptosporidium parvum* - combatting amount of the compound according to Formula (I). Novel compounds useful for treating *Cryptosporidium parvum* are also disclosed.

The foregoing and other objects and aspects of the present invention are explained in detail in the specification set forth hereinbelow.

### Detailed Description of the Invention

The term "loweralkyl", as used herein, refers to C₁-C₆ linear or branched alkyl, such as methyl, ethyl, propyl, butyl, isopropyl, *sec*-butyl, *tert*-butyl, pentyl, isopentyl, and hexyl. Isoalkyl groups, such as isopropyl, isobutyl, isopentyl, and the like are currently preferred. The term "loweralkoxy" or "ocyalkyl" as used herein, refers to C₁-C₆ linear or branched alkoxy, such as methoxy, ethoxy, propyloxy, butyloxy, isopropyloxy, and *t*-butyloxy. Methoxy is currently preferred.

As noted above, the medicaments of the present invention are useful for treating *Pneumocystis carinii* pneumonia, *Giardia lamblia,* and *Cryptosporidium parvum*. The medicaments of the present invention are useful for treating these conditions in that they inhibit the onset, growth, or spread of the condition, cause regression of the condition, cure the condition, or otherwise improve the general well-being of a subject inflicted with, or at risk of contracting the condition.

Subjects to be treated by the medicaments of the present invention are typically human subjects although the medicaments of the present invention may be useful with any suitable subject known to those skilled in the art. As noted above, the present invention provides pharmaceutical formulations comprising the aforementioned compounds of Formula (I), or pharmaceutically acceptable salts thereof, in pharmaceutically acceptable carriers for aerosol, oral, and parenteral administration as discussed in greater detail below. Also, the present invention provides such compounds or salts thereof which have been lyophilized and which may be reconstituted to form pharmaceutically acceptable formulations for administration, as by intravenous or intramuscular injection.

Obviously, the therapeutically effective dosage of any specific compound, the use of which is in the scope of present invention, will vary somewhat from compound to compound, patient to patient, and will depend upon the condition of the patient and the route of delivery. As a general proposition, a dosage from about 0.1 to about 50 mg/kg will have therapeutic efficacy, with still higher dosages potentially being employed for oral and/or aerosol administration. Toxicity concerns at the higher level may restrict intravenous dosages to a lower level such as up to about 10 mg/kg, all weights being calculated based upon the weight of the active base, including the cases where a salt is employed. Typically a dosage from about 0.5 mg/kg to about 5 mg/kg will be employed for intravenous or intramuscular administration. A dosage from about 10 mg/kg to about 50 mg/kg may be employed for oral administration. The duration of the treatment is usually once per day for a period of two to three weeks or until the condition is essentially controlled. Lower doses given less frequently can be used to prevent or reduce the incidence or recurrence of the infection.

In accordance with the present invention, a compound of Formula (I), or a pharmaceutically acceptable salt thereof, may be administered orally or through inhalation as a solid, or may be administered intramuscularly or intravenously as a solution, suspension, or emulsion. Alternatively, the compound or salt may also be administered by inhalation, intravenously or intramuscularly as a liposomal suspension. When administered through inhalation the compound or salt should be in the form of a plurality of solid particles or droplets having a particle size from about 0.5 to about 5 microns, preferably from about 1 to about 2 microns.

Besides providing a medicament for treating *Pneumocystis carinii* pneumonia, the compounds of Formula (I) also provide a medicament for prophylaxis against *Pneumocystis carinii* pneumonia in an immunocompromised patient, such as one suffering from AIDS, who has had at least one episode of *Pneumocystis carinii* pneumonia, but who at the time of treatment is not exhibiting signs of pneumonia. As *Pneumocystis carinii* pneumonia is an especially potentially devastating disease for immunocompromised patients it is preferable to avoid the onset of *Pneumocystis carinii* pneumonia, as compared to treating the disease after it has become symptomatic. Accordingly, the present invention provides a medicament for the prophylaxis against *Pneumocystis carinii* pneumonia wherein said medicament is administered to the patient in a prophylactically effective amount of the compound of Formula (I) or a pharmaceutically acceptable salt thereof. The forms for administration of the compound or salt may be the same as utilized for the purpose of actually treating a patient suffering from *Pneumocystis carinii* pneumonia.

An additional useful aspect of the present invention is a medicament for prophylaxis against even an initial episode of *Pneumocystis carinii* pneumonia in an immunocompromised patient who has never experienced an episode of *Pneumocystis carinii* pneumonia. In this respect, a patient who has been diagnosed as being immunocompromised, such as one suffering from AIDS or ARC (AIDS related complex), even before the onset of an initial episode of *Pneumocystis carinii* pneumonia, may avoid or delay suffering from the infection by having administered a prophylactically effective amount of a compound of Formula (I) or a pharmaceutically acceptable salt thereof. The compound or salt may be administered in the same fashion as in the treatment of patients suffering from *Pneumocystis carinii* pneumonia.

The present invention also provides new pharmaceutical compositions suitable for intravenous or intramuscular injection. The pharmaceutical compositions comprise a compound of Formula (I), or a pharmaceutically acceptable salt thereof, in any pharmaceutically acceptable carrier. If a solution is desired, water is the carrier of choice with respect to water-soluble compounds or salts. With respect to the water-insoluble compounds or salts, an organic vehicle, such as glycerol, propylene glycol, polyethylene glycol, or mixtures thereof, may be suitable. In the latter instance, the organic vehicle may contain a substantial amount of water. The solution in either instance may then be sterilized in any suitable manner, preferably by filtration through a 0.22 micron filter. Subsequent to sterilization, the solution may be filled into appropriate receptacles, such as depyrogenated glass vials. Of course, the filling should be done by an aseptic method. Sterilized closures may then be placed on the vials and, if desired, the vial contents may be lyophilized.

In addition to compounds of Formula (I) or their salts, the pharmaceutical compositions may contain other additives, such as pH adjusting additives. In particular, useful pH adjusting agents include acids, such as hydrochloric acid, bases or buffers, such as sodium lactate, sodium acetate, sodium phosphate, sodium citrate, sodium borate, or sodium gluconate. Further, the compositions may contain microbial preservatives. Useful microbial preservatives include methylparaben, propylparaben, and benzyl alcohol. The microbial preservative is typically employed when the formulation is placed in a vial designed for multidose use. Of course, as indicated, the pharmaceutical compositions of the present invention may be lyophilized using techniques well known in the art.

In yet another aspect of the present invention, there is provided an injectable, stable, sterile composition comprising a compound of Formula (I), or a salt thereof, in a unit dosage form in a sealed container. The compound or salt is provided in the form of a lyophilizate which is capable of being reconstituted with a suitable pharmaceutically acceptable carrier to form a liquid composition suitable for injection thereof into man. The unit dosage form typically comprises from about 10 mg to about 10 grams of the compound or salt. When the compound or salt is substantially water-insoluble, a sufficient amount of emulsifying agent which is physiologically acceptable may be employed in sufficient quantity to emulsify the compound or salt in an aqueous carrier. One such useful emulsifying agent is phosphatidyl choline.

Other pharmaceutical compositions may be prepared from the water-insoluble compounds of Formula (I), or salts thereof, such as aqueous base emulsions. In such an instance, the composition will contain a sufficient amount of pharmaceutically acceptable emulsifying agent to emulsify the desired amount of the compound of Formula (I) or salt thereof. Particularly useful emulsifying agents include phosphatidyl cholines, and lecithin.

Further, the present invention provides liposomal formulations of the compounds of Formula (I) and salts thereof. The technology for forming liposomal suspensions is well known in the art. When the compound of Formula (I) or salt thereof is an aqueous-soluble salt, using conventional liposome technology, the same may be incorporated into lipid vesicles. In such an instance, due to the water solubility of the compound or salt, the compound or salt will be substantially entrained within the hydrophilic center or core of the liposomes. The lipid layer employed may be of any conventional composition and may either contain cholesterol or may be cholesterol-free. When the compound or salt of interest is water-insoluble, again employing conventional liposome formation technology, the salt may be substantially entrained within the hydrophobic lipid bilayer which forms the structure of the liposome. In either instance, the liposomes which are produced may be reduced in size, as through the use of standard sonication and homogenization techniques.

Of course, the liposomal formulations containing the compounds of Formula (I) or salts thereof, may be lyophilized to produce a lyophilizate which may be reconstituted with a pharmaceutically acceptable carrier, such as water, to regenerate a liposomal suspension.

Pharmaceutical formulations are also provided which are suitable for administration as an aerosol, by inhalation. These formulations comprise a solution or suspension of the desired compound of Formula (I) or a salt thereof or a plurality of solid particles of the compound or salt. The desired formulation may be placed in a small chamber and nebulized. Nebulization may be accomplished by compressed air or by ultrasonic energy to form a plurality of liquid droplets or solid particles comprising the compounds or salts. The liquid droplets or solid particles should have a particle size in the range of about 0.5 to about 5 microns. The solid particles can be obtained by processing the solid compound of Formula (I), or a salt thereof, in any appropriate manner known in the art, such as by micronization. Most preferably, the size of the solid particles or droplets will be from about 1 to about 2 microns. In this respect, commercial nebulizers are available to achieve this purpose.

Preferably, when the pharmaceutical formulation suitable for administration as an aerosol is in the form of a liquid, the formulation will comprise a water-soluble compound of Formula (I) or a salt thereof, in a carrier which comprises water. A surfactant may be present which lowers the surface tension of the formulation sufficiently to result in the formation of droplets within the desired size range when subjected to nebulization.

As indicated, the present invention provides both water-soluble and water-insoluble compounds and salts. As used in the present specification, the term "water-soluble" is meant to define any composition which is soluble in water in an amount of about 50 mg/ml, or greater. Also, as used in the present specification, the term "water-insoluble" is meant to define any composition which has solubility in water of less than about 20 mg/ml. For certain applications, water soluble compounds or salts may be desirable whereas for other applications water-insoluble compounds or salts likewise may be desirable.

The compounds of Formula (I) above which are particularly preferred for *Pneumocystis carinii* pneumonia, *Giardia lamblia*, and *Cryptosporidium parvum* include a variety of compounds. For example, particularly preferred compounds are defined by Formula (I) wherein X and Y are each 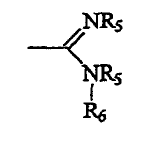 and (a): R₁ is H, R₂ is H, R₃ is H, R₄ is H, R₅ is H, and R₆ is H, C₁₋₆ alkyl, hydroxy C₁₋₆ alkyl, amino C₁₋₆ alkyl, C₁₋₆ alkylamino, or C₁₋₆ alkylamino C₁₋₆ alkyl; (b): R₁ is H or C₁₋₆ alkyl, R₂ is C₁₋₆ alkyl, R₃ is H, R₄ is H, R₅ is H, and R₆ is H or C₁₋₆ alkyl; (c): R₁ is H or oxy C₁₋₆ alkyl, R₂ is H, or oxy C₁₋₆ alkyl, R₃ is H, R₄ is H, two R₅ groups together represent C₂ alkyl, and R₆ is H, C₁₋₆ alkyl or hydroxy C₁₋₆ alkyl; (d): R₁ is H, R₂ is H, R₃ is H, R₄ is H, two R₅ groups together represent C₃ alkyl, and R₆ is H; (f): R₁ is H, R₂ is H, R₃ is H, R₄ is H, two R₅ groups together represent 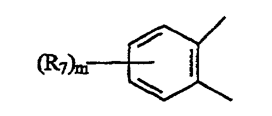 wherein m is 1, and R₇ is H or -CONHR₈NR₉R₁₀, wherein R₈ is C₁₋₆ alkyl, and R₉ and R₁₀ are each H, and R₆ is H; and (e): R₁ is H, R₂ is H, R₃ is H, R₄ is H, two groups R₅ groups together represent C₄ alkyl, and R₆ is H.

Examples of compounds exemplary of Formula (I) above include, but are not limited to:
2,5-bis(4-amidinophenyl)furan,
2,5-bis(4-amidinophenyl)-3,4-dimethylfuran,
2,5-di-p[2(3,4,5,6-tetrahydropyrimidyl)phenyl]furan,
2,5-bis[4-(2-imidazolinyl)phenyl]furan,
2,5-[bis{4-(2-tetrahydropyrimidinyl)}phenyl]3-p(tolyloxy)furan,
2,5-[bis{4-(2-imidazolinyl)phenyl]3-p(tolyloxy)furan,
2,5-bis{4-[5-(N-2-aminoethylamido)benzimidazol-2-yl]phenyl}furan,
2,5-Bis[4-(3a,4,5,6,7,7a-hexahydro-1H-benzimidazol-2-yl)phenyl]furan,
2,5-bis[4-(4,5,6,7-tetrahydro-1H-1,3-diazepin-2-yl)phenyl]furan,
2,5-bis(4-N,N-dimethylcarboxhydrazidephenyl)furan,
2,5-bis{4[2-(N-2-hydroxyethyl)imidazolinyl]-phenyl}furan,
2,5-bis[4-(N-isopropylamidino)phenyl]furan,
2,5-bis{4-[3-(dimethylaminopropyl)amidino]phenyl}furan,
2,5-bis-{4-[N-(3-aminopropyl)amidino]phenyl}furan,
2,5-bis[2-(imidazolinyl)phenyl]-3,4-bis(methoxymethyl)furan,
2,5-bis[4-N-(dimethylaminoethyl)amidino]phenylfuran,
2,5-bis-{4-[(N-2-hydroxyethyl)amidio]phenyl}furan,
2,5-bis-[4-N-(cyclopropylamidino)phenyl]furan,
2,5-bis-[4-(N,N-diethylaminopropyl)amidino]phenyl furan,
2,5-bis-{4-[2-(N-ethylimidazolinyl)]phenyl}furan,
2,5-bis-{4-[N-(3-pentylamidino)]}phenylfuran,
2,5-bis-[4-(2-imidazolinyl)phenyl]-3-methoxyfuran,
2,5-bis[4-(N-isopropylamidino)phenyl]-3-methylfuran,
and the pharmaceutically acceptable salts thereof.

Compounds employed in carrying out the present invention may be prepared in accordance with techniques known to those skilled in the art (see, e.g., B.P. Das, et al., Synthesis and antiprotozoal activity of 2,5-Bis(4-amidinophenyl) furans, *J. Med. Chem.* **20**:531 (1977), the disclosure of which is incorporated herein by reference in its entirety), particularly in light of the disclosure and examples set forth below.

As indicated, the compounds used in the present invention may be present as pharmaceutically acceptable salts. Such salts include the gluconate, lactate, acetate, tartarate, citrate, phosphate, borate, nitrate, sulfate, and hydrochloride salts.

The salts of the present invention may be prepared, in general, by reacting two equivalents of the pyrimidine base compound with the desired acid, in solution. After the reaction is complete, the salts are crystallized from solution by the addition of an appropriate amount of solvent in which the salt is insoluble.

Pharmaceutical formulations of the compounds of Formula (I) for combating *Giardia lamblia* are prepared in essentially the same manner as given above.

Pharmaceutical formulations of the compounds of Formula (I) for combating *Cryptosporidium parvum* are prepared in essentially the same manner as given above.

The compounds of the present invention are useful not only for treating *Pneumocystis carinii* pneumonia, *Giardia lamblia*, and *Cryptosporidium parvum*, but also in inhibiting enzymes such as topoisomerase. The compounds of Formula (I) are particularly useful for inhibiting topoisomerase II. *See,* S. Doucc-Racy, et al., *Proc. Natl. Acad. Sci. USA* **83**:7152 (1986).

As noted above, the compounds useful in the present invention may be prepared according to techniques known in the art. According to one method, the compounds of Formula (I) can be prepared by: (a) cyclodehydrative furanization of 1,4-diketones according to the procedure taught by R.E. Lutz, et al., *J. Am. Chem. Soc.* **56**:2698 (1934) to form 2,5-bis-(4-bromophenyl) furan; (b) nitrilization of 2,5-bis(4-bromophenyl) furan using copper (I) cyanide to produce the corresponding bis-nitrile 2,5-bis-(4-cyanophenyl) furan; and (c) conversion of the bis-nitrile to the desired bis-dicationic aryl furan of Formula (I). This method is illustrated in **Scheme 1** below.

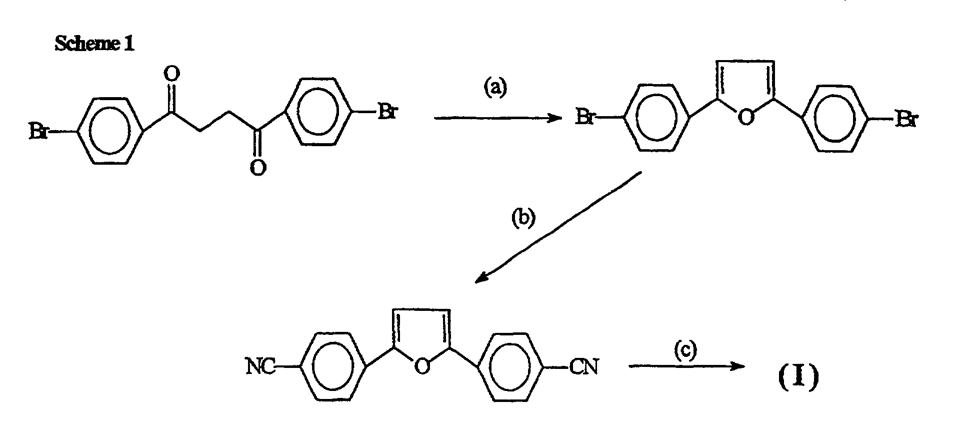

According to a second method, compounds of Formula (I) may be prepared by (a) converting the appropriate bromoacetophenone to the ethyl bromophenyl-oxopropionate using sodium hydride and diethylcarbonate in tetrahydrofuran, (b) converting the ethyl bromophenyl-oxopropionate to the ethyl bis-bromobenzoylpropionate using bromophenacyl bromide, (c) converting the bis-bromobenzoylpropionate to the ethyl bis-bromophenyl furan using ethanol and hydrochloric acid, (d) hydrolysis of the ethyl bis-bromophenyl furan to the bis-bromophenyl furan carboxylic acid using potassium hydroxide followed by hydrochloric acid, (e) converting the carboxylic acid to the corresponding bis-nitrile with copper (I) cyanide and heat, and converting the bis-nitrile to the appropriate bis-dicationic aryl furan.
This method is illustrated in **Scheme 2** below.

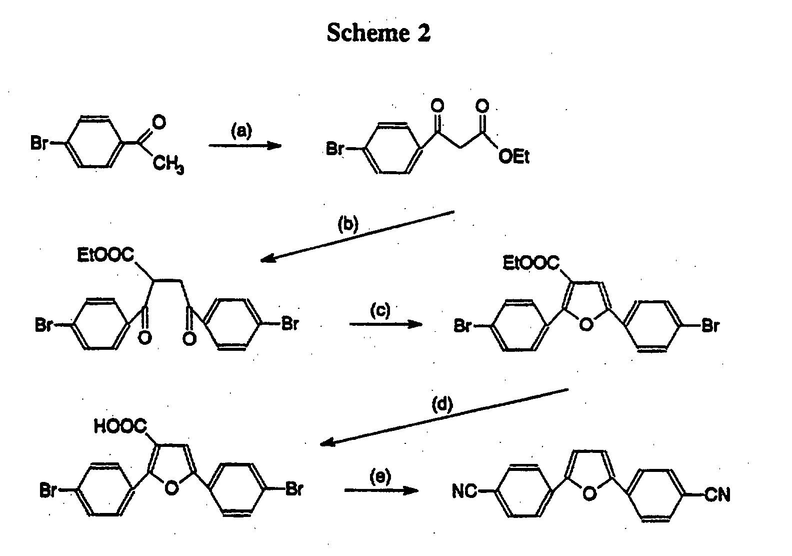

Conversion of the bis-nitrile to the bis-dicationic aryl furan of Formula (I) may be accomplished according to several methods known to those skilled in the art. According to one currently preferred method, conversion of the bis-nitrile to the bis-dicationic aryl furan is carried out by conversion into intermediate imidate esters using classical Pinner methodology, followed by reaction of these intermediates with ammonia or the appropriate diamine for example, ethylenediamine, 1,3-propanediamine, etc., as exemplified in the Examples set forth below. According to a second currently preferred method, the bis-nitrile is converted to the bis-dicationic aryl furan by fusion of the bis-nitrile directly with the hydrochloride salt of the appropriate diamine by thermolysis. This technique is particularly useful for the preparation of compounds wherein two R₅ groups together form a cyclic alkyl.

The present invention is explained in greater detail in the following examples. As used herein,, "mp" means melting point, "NMR" means nuclear magnetic resonance, "MHz" means megahertz, "FAB" means fast atomic bombardment, "EI" means electron ionization, "IR" means infrared spectra, "MS" means mass spectroscopy, "Hz" means hertz, "g" means grams, "ml" means milliliters, "L" means liters, "hr" means hours, "°C" means degrees Centigrade, "DMSO" means dimethyl sulfoxide, "DMF" means dimethyl formamide, and "m/e" means mass divided by charge. These Examples are illustrative and are not to be taken as limiting of the invention.

Melting points are recorded using a Thomas Hoover (Uni-Melt) capillary melting point apparatus and are uncorrected. ¹H NMR and ¹³C NMR spectra are recorded employing a Varian GX400 spectrometer and chemical shifts (δ) are in ppm relative to TMS unless otherwise noted. Mass spectra are recorded on a VG Instruments 70-SE spectrometer. IR spectra are recorded using a Michelson 100 instrument.

### EXAMPLE 1

### Preparation of Precursor Compounds

**2,5-Bis(p-bromophenyl)furan.** A literature procedure as known in the art for preparation of trans-di-p-bromobenzoylethylene from bromobenzene and fumaryl chloride was employed. J.B. Conant and R.E. Lutz, *J.Am. Chem. Soc.* **47**, 881 (1925). The ethylene compound was reduced with Zn-HOAc to prepare 1,4-di-p-bromophenyl-1,4-butanedione. E. Campaigne and W.O. Foye, *J*.*Org*.*Chem*. **17**, 1405 (1952). The saturated 1,4-diketone (7.9 g, 0.02 mol) was suspended in 80 ml of AC₂O and the mixture was heated to reflux. Concentrated H₂SO₄ (4-5 drops) was added and refluxing was continued for 5 min. The solution was poured into water-ice (1 L), stirred well, and filtered: crude yield 7 g (93%). Recrystallization from acetic acid gave 5.6 g (75%), mp 198-199°C (lit. (R.E. Lutz and W.M. Eisner, *J.Am.Chem.Soc*. **56,** 2698 (1934)) mp 200-201 °C).

**2,5-Bis(p-cyanophenyl)furan**. A mixture of 7.5 g (0.02 mol) of 2,5-bis-(4-bromophenyl)furan and 4 g (0.045 mol) of Cu(CN) in 45 ml of quinoline was refluxed for 2 h. The mixture was poured into 300 ml of dilute HCl solution (caution, HCN is liberated) and filtered. The solid was washed with H₂O, dilute NaOH, dilute HCI, and again with H₂O. The solid bis-nitrile was dissolved in acetone, filtered to remove inorganic residue, and passed through a short alumina column to remove traces of copper salts. The copper salts must be removed since they carry over to the bis-amidines from which they are difficult to purify. A convenient method to detect the presence of copper salts is a flame test. Evaporation of the eluent from the alumina column and recrystallization from ethanol gave 3.5 g (65%), mp 294-295°C.

### EXAMPLE 2

### Preparation of 2.5-Bis(4-amidinophenyl)furan dihydrochloride

2,5-Bis(4-cyanophenyl)furan (3 g, 0.011 mol) (prepared as described in **Example 1**) in a mixture of 100 ml of dioxane and 25 ml of absolute ethanol was saturated with dry HCl gas at 5°C. The solution was placed in a pressure bottle and shaken for 3 days (room temperature). An intermediate product, an imidate ester hydrochloride, precipitated as a yellow solid, was filtered and dried under vacuum at room temperature overnight. The IR spectra of the imidate ester hydrochloride was free of adsorption for nitrile and it was used directly without further characterization. A suspension of the imidate ester hydrochloride (3.5 g) in 100 ml of absolute ethanol was saturated at 5°C with anhydrous ammonia. The suspension (pressure bottle) was shaken for 3 days at room temperature. The reaction mixture was filtered and the solid was dried and dissolved in warm absolute ethanol (ca. 1. 5 L). The solution was acidified with anhydrous HCl at 5°C, concentrated under vacuum at room temperature, and 2.5 g (60%) of yellow crystals were obtained. Recrystallization from absolute ethanol gave mp 400-401°C dec.

### EXAMPLE 3

### Preparation of 2,5-Bis[4-(4,5-dihydro-1H-imidazol-2-yl) phenyl] furan

A solution of an imidate ester hydrochloride intermediate synthesized as described in **Example 2**, 2.1 g (0.005 mol), and 0.6 g (0.01 mol) of ethylenediamine in 50 ml of absolute ethanol was refluxed overnight. The solid which formed was filtered and recrystallized from absolute ethanol saturated with anhydrous HCl to yield 2,5-Bis[4-(2-imidazolinyl)phenyl]furan, 1.9 g (90%), mp 409-410°C dec.

### EXAMPLE 4

### Preparation of 2,5-Bis[4-(4,5-dihydro-1H-imidazol-2-yl)phenyl]furan dihydrochloride dihydrate

Bis-nitrile (0.5g, 1.9 mmole), ethylenediamine dihydrochloride (4.9 g, 37 mmole), ethylenediamine (2.5 ml, 37 mmole) are mixed. The mixture is heated at 300-310°C for 10 minutes in a sand bath. After cooling the mixture is dissolved in hot water. Yellow crystals separate on cooling. The compound is recrystallized from boiling water to yield 208 mg (24%), and then dried under vacuum at 80°C for 12 hr. TLC (CHCl₃:CH₃OH:25% NH₄OH = 11:4:1, one spot), mp>360°C. Analysis calculated for C₂₂H₂₀N₄O·2HCl·2H₂O: C:56.78, H:5.60, N:12.04; found: C:56.69, H:5.63, N:12.07. ¹H-NMR (DMSO-d₆, TMS), δ 4.01 (s, 8H), 7.45 (s, 2H), 8.08 (d, 4H, J=8.3 Hz), 8.15 (d, 4H, J=8.3 Hz), 8.15 (d, 4H, J=8.3 Hz), 10.50 (brs, 4H). ¹³C-NMR (DMSO-d₆, TMS), δ 45.5, 113.2, 121.6, 125.3, 130.1, 135.8, 141.3, 153.4, 165.8. IR (KBr): 3412, 3123, 2971, 1608, 1580, 1491, 1367, 1287, 1033, 850, 745, 673 cm⁻¹. MS m/e 356 (free base).

### EXAMPLE 5

### Preparation of 2,5-Bis[4-(1,4,5,6-tetrahydropyrimidin-2-yl) phenyl] furan

In a similar manner as set forth in **Example 3**, an imidate ester hydrochloride synthesized as described above in **Example 2** was reacted with 1,3-propanediamine to yield (90%) of the 2,5-Bis[4-(1,4,5,6-tetrahydropyrimidin-2-yl)phenyl] furan, mp 430-431°C dec.

### EXAMPLE 6

### Preparation of 2,5-Bis[4-(4,5,6,7-tetrahydro-1H-1,3-diazepin-2-yl) phenyl]furan dihydrochloride

The bis-methoxyethanol imidate ester (1 g, 0.002 mole), 1,4-diaminofurane (0.5 g) in 10 ml of 1,2-dimethoxyethane are refluxed for 2 days. The solvent is removed under vacuum and water is added. The precipitate is filtered, washed with water, and dried in vacuum oven (mp > 300°C). The filtrate is neutralized using 2 M sodium hydroxide and another portion of the free base is obtained. The crude free base is converted into the hydrocholride (mp > 300°C) by hydrogen chloride in methanol. The total yield of free base is 51%. Analysis calculated for C₂₆H₂₈N₄O·2HCl·3.5H₂O (548.50): C:56.93, H:6.80, N: 10.22; found: C:56.99, H:6.80, N:10.26. ¹H-NMR (DMSO-d₆) δ 2.02 (s, 8H), 3.71 (s, 8H), 7.38 (s, 2H), 7.86 (d, 4H, J=8.3 Hz), 8.04 (d, 4H, J=8.3 Hz), 9.77 (s, 4H). ¹³C-NMR (D₂O (CH₃)₃SiCH₂CH₂CO₂Na), δ 28.0, 47.0, 113.9, 126.5, 129.7, 131.2, 137.0, 154.5, 167.1. IR (KBr), 687, 747, 814, 930, 1131, 1364, 1459, 1597, 3008, 3164 cm⁻¹. MS (EI) m/e 412 (free base).

### EXAMPLE 7

### Preparation of 2,5-Bis[4-(3a,4,5,6,7,7a-hexahydro-1H-benzimidazol-2-yl)phenyl]furan dihydrochloride

1,2-Diaminocyclohexane (9 ml) is treated with ethanolic HC. The solution is evaporated to dryness and a new portion of the amine (9 ml), and 2,5-bis-(4-cyanophenyl)furan (2 g) is added. The mixture is maintained at 300-310°C in a sand bath for about 10 min. Progress of the reaction is monitored by TLC (CHCl₃:CH₃OH:NH₄OH=44:8:1, v | v | v). After cooling the residue is recrystallized from water to afford analytically pure yellow crystals (1.4 g, 36%), having an mp>300°C. Analysis calculated for C₃₀H₃₂N₄O·2HCl·0.25H₂O: C:66.48, H:6.42, N: 10.34; found: C:66.47, H:6.40, N: 10.30. ¹H-NMR (DMSO-d₆, TMS), δ 1.30-2.00 (m, 16H), 4.36 (s, 4H), 7.46 (s, 2H), 8.18 (m, 8H), 10.87 (s, 4H). ¹³C-NMR (DMSO-d₆, D₂O) δ 18.9, 25.8, 57.0, 112.7, 122.3, 125.0, 130.0, 135.5, 153.4, 164.9. IR (KBr) 3413, 2991, 1597, 1501, 1354, 1293, 1016, 930, 853, 796, 747, 676 cm⁻¹. MS m/e 464 (free base).

### EXAMPLE 8

### Preparation of 2,5-Bis[4-(4,5-dihydro-1-(hydroxyethyl)-1H-imidazol-2-yl)phenyl] furan dihydrochloride dihydrate

A mixture of N-(2-hydroxyethyl)ethylene diamine (0.64g, 0.006 mole) in 15 ml absolute ethanol and the imidate ester (0.87 mole, 0.002 mole) is heated under reflux for 12 hr. The solvent is removed by distillation, and the resulting residue is triturated with ice/water, the pH is adjusted to 10 with 2 M NaOH and the precipitated solid is filtered, washed with water, dried, and recrystallized from ethanol-ether to yield a light yellow crystalline solid 0.72g (81%) having an mp of 119-120°C. IR(KBr) 3390, 3290, 3132, 2864, 1615, 1595, 1421, 1276, 1059, 849 cm⁻¹. ¹H-NMR (DMSO-d₆) δ 7.87 (d, 4H, J=8.3), 7.64 (d, 4H, J=8.3), 7.16 (s, 2H), 3.75 (t, 4H, J=9.8), 3.53 (t, 4H, J=5.8), 3.47 (t, 4H, J=9.8), 3.1 (t, 4H, J=5.8). ¹³C-NMR (DMSO-d₆) δ 165.8, 152.4, 130.8, 128.7, 123.1, 109.2, 59.3, 52.6, 51.4, 51.1. MS m/e 444.

The free base 0.45 g (0.001 mole) is suspended in 10 ml ethanolic HCl and heated under reflux for 30 min, concentrated in vacuum, and triturated with dry ether to yield a shining yellow crystalline. The solid is filtered, washed with ether, and dried in vacuum to yield 0.45 g (89%) having an mp 178-179°C. IR(KBr) 3407, 3089, 2919, 1615, 1569, 1370, 1288, 1067, 853, 669 cm⁻¹. ¹H-NMR (DMSO-d₆/50°C) δ 10.8 (brs, 2H), 8.9 (d, 4H, J=8.6), 7.87 (d, 4H, J=8.6), 7.4 (s, 2H), 5.45 (vbr, 2H), 4.18-4.10 (m, 4H), 3.99-3.92 (m, 4H), 3.66-3.5 (m, 4H), 3.48-3.42 (m, 4H). ¹³C-NMR (DMSO-d₆/50°C) δ 165.9, 152.3, 133.4, 129.8, 123.7, 121.5, 111.2, 56.6, 49.4, 49.2, 42.5. Analysis calculated for C₂₆H₂₈N₄O₃·2HCl·H₂O: C:61.80, H:6.38, N: 11.09; found: C:61.62, H:6.51, N:10.89.

### EXAMPLE 9

### Preparation of 2,5-Bis[4-(4,5-dihydro-1-(ethyl)-1H-imidazol-2-yl)phenyl] furan dihydrochloride dihydrate

To a suspension of the imidate ester (0.65 g, 0.0015 mole) in 10 ml absolute ethanol is added N-ethylethylene diamine (0.4 g, 0.0045 mole), and the mixture is heated at reflux under nitrogen for 12-14 hr. The solvent is removed by distillation under vacuum, the remaining oil was diluted with ice-water and basified with 1 M NaOH to pH 10. A gummy solid separates from the aqueous phase. The solid is washed with water, dried in vacuum, and recrystallized from ethanol:CHCl₃ to yield a hygroscopic yellow solid, 0.45 g (73%). MS m/e 412. The free base (0.41 g, 0.001 mole) is dissolved in 10 ml ethanolic HCl and stirred at 35-40°C for 1 hr. The excess solvent is distilled under vacuum and the resulting semi-solid was triturated with dry ether. The ether was removed under vacuum and the resulting solid was dried under vacuum to yield a very hygroscopic yellow crystalline solid 0.45 g (93%) having an mp 163-4°C. ¹H NMR (D₂O/DMSOd₆) δ 8.08 (d, 4H, J=8.3), 7.76 (d, 4H, J=8.3), 7.26 (s, 2H), 4.24-4.06 (m, 8H), 3.6-3.56 (m, 4H), 1.34 (brt, 6H). ¹³C NMR (D₂O/DMSOd₆) δ 165.0, 154.2, 133.5, 129.3, 123.9, 121.3, 111.3, 48.9, 42.5, 41.7, 12.4. Analysis calculated for C₂₆H₂₈N₄O·2HCl·H₂O: C:61.17, H:6.32, N:10.97; found: C:60.93, H:6.44, N:10.85.

### EXAMPLE 10

### Preparation of 2,5-Bis{[4-(N-isopropyl)-amindino] phenyl} furan

Dry isopropylamine (0.47 g, 0.008 mole) was added to a suspension of an imidate ester as described in **Example 2** (1.3 g, 0.003 mole) in 45 ml absolute ethanol. Within 0.5 hr the imidate ester dissolved and the mixture of the imidate ester and isopropylamine became colored. After ca. 3 hr a white solid precipitated; the slurry was stirred overnight at room temperature. The solvent was removed under reduced pressure, diluted with water, filtered and washed with water. After the solid was dried, it was recrystallized from an ethanol/ether mixture to yield a white solid 0.9 g (78%); mp 233-4°C, ¹H NMR (DMSO-d₆)/60°C) 7.79 (brs, 8H), 7.11 (s, 2H), 6.25 (br, 4H, 3.81 (br, 2H), 1.14 (d, 6H, J=5.9). ¹³C NMR (DMSO-d₆/60°C) 152.4, 142.0, 136.6, 130.4, 126.8, 122.8, 108.7, 43.5, 22.8.

### EXAMPLE 11

### Preparation of 2,5-Bis[4-N-isopropyl)amindino) phenyl] furan dihydrochloride

The free base (0.78 g, 0.002 mole) prepared as described in **Example 10** was dissolved in 10 ml absolute ethanol and treated with 10 ml of ethanol saturated with hydrogen chloride and warmed for 2 hr. The mixture was reduced in volume to 5 ml. Addition of 20 ml of dry ether produced a bright yellow precipitate which was filtered, washed with 3x5 ml dry ether and dried in vac. at 65°C for 2 hr to yield 0.8 g (87%). Mp 276-7°C(dec). IR (KBr). ¹H NMR (DMSO-d₆) 9.72 (s, 1H) 9.69 (s, 1H), 9.57 (s, 2H), 9.24 (s, 2H), 8.06 (d, 4H, J=8.1), 7.86 (d, 4H, J=8.1), 7.42 (s, 2H), 4.14 (s, 2H, J=6.6), 1.29 (d, 12H, J=6.6). ¹³C NMR (DMSO-d₆) 161.1, 152.3, 133.6, 129.2, 127.7, 123.5, 111.3, 45.1, 21.1.

Anal. Calculated for: C₂₄H₂₈N₄O·2HCl·1.25 H₂O: C, 59.57; H, 6.79; N, 11.57. Found: C, 60.00; H, 6.80; N, 11.52.

### EXAMPLE 12

### Preparation of 2,5-bis[(4-(4,5-dihydro-1H-imidazol-2-yl) phenyl)]-3-(4-tolyloxy) furan

**1-(4-tolyloxyl)-1,2-bis(4-bromobenzoyl)ethylene.** To a solution of 1,2-dibromo-1,2-di(4-bromobenzoyl) ethane (11.1 g, 0.02 mole) in 35 ml of THF was added a suspension of sodium 4-methyl phenoxide [prepared from 0.92 g (0.04 mole) Na and 4.32 g (0.04 mole) 4-methylphenol in 30 ml THF by refluxing for 4-5 hr]. The yellow mixture was refluxed for 2-3 hr (TLC followed) after which the THF was removed under reduced pressure. The residue was treated with water, and the solid was filtered, washed with water, dried (Na₂SO₄), and dissolved in chloroform. The chloroform solution was passed through a silica column (elution with 2-5% ether in hexane). The result was an off white crystalline solid, 4.95 g (50%), mp 137-8°C. IR (KBr) 3087, 3035, 2868, 1687, 1646, 1587, 1572, 1557, 1502, 1399, 1364, 1194, 1068, 1009, 971, 876, 815, 772, 526. ¹H NMR (CDCl₃/35°C) 7.92 (d, 2H, J=8.8), 7.65 (d, 2H, J=8.8), 7.55 (d, 2H, J=8.8), 7.48 (d, 2H, J=8.8), 7.27 (d, 2H, J=8.3), 7.11 (d, 2H, J=8.3), 6.32 (s, 1H), 2.4 (s, 3H). ¹³C NMR (CDCl₃/35°C) 189.4, 187.6, 168.4, 150.9, 136.6, 136.0, 133.4, 132.3, 131.8, 130.9, 130.3, 129.6, 129.2, 128.2, 120.6, 101.8, 20.95. MS m/e 500 (M⁺).

**2,5-bis(4-Bromophenyl)-3-(p-tolyloxy) furan.** A solution of 5.0 g (0.01 mole) 1-(4-tolyloxy)-1,2-bis-(4-bromobenzoyl)ethylene in 10 ml phosphorus trichloride was heated under reflux for 3-4 hr (TLC followed). The excess PCl₃ was removed by distillation and the residue was triturated with ice/water (exothermic reaction). The solution was extracted with dichloromethane (75 ml) and the dichloromethane layer was washed with saturated sodium bicarbonate solution, water, and dried (Na₂SO₄). The solvent was removed under reduced pressure. The residual solid was chromatographed over silica gel using ether:hexane (2:8 to 1:1) as eluant. An off white crystalline solid was obtained, 2.78 g (56%), mp 92-3°C. IR (KBr) 2923, 2851, 1560, 1506, 1467, 1390, 1209, 1072, 1066, 945, 825, 707, 486. ¹H NMR (CDCl₃/35°C) 7.69 (d, 2H, J=8.8), 7.46-7.43 (m, 6H), 7.12 (d, 2H, J=8.3), 7.0 (d, 2H, J=8.3), 6.47 (s, 1H), 2.31 (s, 3H). ¹³C NMR (CDCl₃/135°C) 150.8, 150.1, 142.8, 139.3, 133.0, 131.9, 131.7, 130.3, 129.1, 128.6, 125.1, 125.0, 121.8, 120.5, 117.1, 102.7, 20.6. MS m/e 484 (M⁺).

**2,5-bis(4-Cyanophenyl)-3-(4-tolyloxy) furan.** A mixture of the dibromo compound prepared above (2.5 g, 0.0051 mole) and cuprous cyanide (1.81 g, 0.02 mole) in 8 ml dry N-methyl-2-pyrrolidone was heated at ca. 200°C under a nitrogen atmosphere for 2.5 hr (TLC followed), cooled, and poured into 200 ml of water. The precipitated solid was filtered, resuspended in 100 ml of water and 100 ml of 10% NaCN was. added and the mixture was stirred for 3-4 hr. The solid was filtered, washed with water and placed in a soxlate device using acetone for ca. 24 hr. The acetone extract was reduced in volume and passed through a short column of neutral aluminum, the eluate was evaporated and the resulting solid was recrystallized from CHCl₃:ether (2:8) to give a yellow crystalline solid 1.2 g (62%), mp 198-9°C. IR (KBr) 3067, 2223, 1618, 1303, 1505, 1402, 1220, 1169, 1008, 926, 840, 820, 668, 546 cm⁻¹. ¹H NMR (CDCl₃/35°C) 7.98 (d, 2H, J=8.8), 7.75 (d, 2H, 8.3), 7.68 (d, 2H, J=8.8), 7.65 (d, 2H, J=8.8), 7.19 (d, 2H, J=8.3), 7.05 (d, 2H, J=8.3), 6.66 (s, 1H), 2.36 (s, 3H). ¹³C NMR (CDCl₃/35°C) 154.3, 150.3, 145.8, 139.1, 134.0, 133.6, 133.3, 132.7, 132.6, 130.5, 124.2, 123.8, 119.0, 118.6, 117.8, 111.5, 110.0, 104.5, 20.7. Anal. Calcd. for C₂₅H₁₆N₂O₂: C, 79.76; H, 4.28; N, 7.44; Found: C, 79.68; H, 4.31; N, 7.39. MS m/e 376 (M⁺).

**2,5-bis[(4-(4,5-dihydro-lH-imidazol-2-yl)phenyl)]-3-(4-tolyloxy) furan.** The bis-nitrile prepared above [1 g (0.0026 mole)] was placed in 20 ml absolute ethanol and 50 ml absolute dioxane which was saturated with dry HCl gas at 0°C. The mixture was allowed to stir at room temperature for 4 days. A thick yellow precipitate formed, 100 ml of dry ether was added and the solid was filtered, washed with 100 ml dry ether and dried in vacuo at 25 °C for 5 hr to yield 0.78 g (66%) imidate ester hydrochloride. The imidate ester was resuspended into 25 ml dry ethanol and heated at gentle reflux with 0.31 g (0.0053 mole) ethylenediamine for 12 hr. The excess ethanol was removed by distillation and the residue was treated with water, basified with 1 M NaOH (stirring and cooling). The yellow precipiate was filtered, washed with water, dried and recrystallized from boiling ethanol to yield 0.6 g (74%), mp 156-7°C. IR (KBr) 3218, 2927, 2862, 1609, 1506, 1398, 1218, 1105, 987, 848, 669 cm⁻¹. ¹H NMR (DMSO-d₆/50°C) 7.94-7.84 (m, 8H), 7.21 (d, 2H, J=8.3), 7.12 (s, 1H), 7.08 (d, 2H, J=8.79), 3.63 (s, 4H), 3.62 (s, 4H), 2,28 (s, 3H). ¹³C NMR (DMSO-d₆/50°C) 163.0, 162.9, 154.3, 150.4, 142.8, 139.0, 132.4, 130.8, 130.4, 130.1, 129.7, 128.5, 127.5, 127.4, 123.2, 122.6, 116.5, 104.0, 49.3, 49.2, 19.9. MS m/e 462 (M⁺).

The free base [0.5 g (0.001 mole)] in 10 ml ethanolic HCl was heated at reflux 3 hr and added to diluted 50 ml dry ether. The resulting yellow precipitate was filtered, washed with dry ether and dried in vacuo at 80°C for 24 hr, 0.48 g (90%), mp >300°C. Anal. Calculated for C₂₉H₂₆N₄O₂ • 2 HCl: C, 65.04; H, 5.27; N, 10.46. Found C, 64.83; H, 4.99; N. 10.22. IR (KBr) 3422, 3235, 2964, 2775, 1609, 1506, 1370, 1289, 1206, 848, 667 cm⁻¹. ¹H NMR (DMSO-d₆/D₂O/TSP/60°C) 7.98-7.86 (m, 8H), 7.19 (d, 2H, J=8.79), 7.09 (s, 1H), 7.03 (d, 2H, J=8.3), 3.88 (s, 4H), 3.76 (s, 4H), 2.24 (s, 3H). ¹³C NMR (DMSO-d₆/D₂O/TSP/60°C) 165.3, 165.3, 154.7, 151.2, 145.7, 139.5, 134.3, 134.2, 135.1, 131.2, 129.6, 129.5, 124.8, 124.1, 123.3, 121.6, 117.7, 106.0, 45.8, 45.6, 20.7.

### EXAMPLE 13

### Preparation of 2,5-Bis[4-(2-tetrahydro-pyrimidinyl)phenyl]-3-(4-tolyoxy)furan

A stirred mixture of imidate ester (1.08 g, 0.002 mole) and freshly distilled 1,3-diaminopropane (0.43 g, 0.006 mole) in 30 ml absolute ethanol was gently heated under reflux (protected from moisture) for 12 hr. The excess ethanol was removed under reduced pressure and the residue titrated with 50 ml distilled water. The mixture was made basic with 1 M NaOH (pH 10) while cooling and stirring; the precipitated free base was filtered, washed with water, dried and recrystallized from hot ethanol to yield 0.80 g (81.6%); mp 190-191°C. IR (KBr): 3267, 2931, 2858, 1609, 1505, 1369, 1216, 846, 666 cm⁻¹. ¹H NMR (DMSO-d₆/50°C) 7.88-7.78 (m, 8H), 7.2 (d, 2H, J=8.8), 7.12 (s, 1H), 7.07 (d, 2H, J=8.8), 3.38 (t, 8H, J=5.1). 2.28 (s, 3H), 1.75 (tt, 4H, J=5.1): ¹³C NMR (DMSO-d₆/50°C) 154.4, 153.8, 153.4, 150.5, 142.8, 139.0, 134.5, 132.9, 132.4, 130.6, 130.3, 130.3, 126.8, 126.7, 123.1, 122.5, 116.6, 104.1, 41.0, 40.8, 20.0, 19.8; MS m/e 490 (M+).

A suspension of 0.5 g (0.001 mole) of the free base in 5 ml absolute ethanol was treated with 10 ml ethanolic HCl and heated under gentle reflux for 2 hr. 50 ml of dry ether was added and the yellow precipitate thus obtained was filtered and washed with dry ether and dried *in vacuo* at 60°C for 12 hr. The yield of yellow solid 0.46 g (82%). Mp > 320°C. IR(KBr): 3423, 3117, 3002, 1638, 1609, 1507, 1375, 1315, 1202, 846, 669 cm⁻¹; ¹H NMR (DMSO-d₆/D₂O/TSP/65°C) 8.12 (d, 2H, J=7.8), 8.08 (d, 2H, J=7.3), 7.88 (d, 4H, J=8.3), 7.32 (d, 2H, J=8.3), 7.22 (s, 1H), 7.16 (d, 2H, J=8.3), 3.6 (br m, 8H), 2.37 (s, 3H), 2.1 (br m, 4H). ¹³C NMR (DMSO-d₆/D₂O/TSP/65°C): 159.5, 154.8, 151.1, 145.1, 140.9, 139.6, 134.1, 133.9, 133.5, 133.2, 128.7, 128.5, 127.2, 124.8, 117.6, 105.9, 41.5, 41.4, 20.6, 18.2. Anal. calculated for: C₃₁H₃₀N₄O₂•2HCl. C, 66.06; H, 5.36; N, 9.94. Found: C, 65.91; H, 5.21; N, 9.88.

### EXAMPLE 14

### Preparation of 2,5-Bis[4-(2-imidazolinyl)phenyl]-3-methoxy furan

**1,2-Bis(4-bromobenzoyl)-1-methoxyethane.** To a solution of 1,2-dibromo-1,2-di(4-bromobenzoyl) ethane (11.1 g, 0.02 mole) in 150 ml dry methanol was added a solution of sodium methoxide in methanol (0.92 g sodium in 50 ml methanol). The yellow brown mixture was refluxed for 1-1.5 hr. The solvent was removed by distillation, the residue was suspended in water and the mixture was extracted with 100 ml chloroform. The chloroform extract was washed with water, dried (Na₂SO₄) and concentrated. The residue obtained was titrated with dry methanol-ether (3:1) to yield off-white crystalline solid, 6.6 g (78%), mp 153-154°C. IR (KBr): 3106, 3062, 2932, 1689, 1649, 1583, 1556, 1403, 1223, 1202, 1182, 1086, 1010, 1000, 857, 814, 738, 618, 472 cm⁻¹. ¹H (DMSO-d₆/40°C): 7.95 (d, 2H, J=7.8), 7.77 (4H, J=8.8), 7.72 (d, 2H, J=7.8), 6.89 (s, 1H), 4.03 (s, 3H). ¹³C (DMSO-d₆/40°C): 189.9, 187.2, 168.8, 139.9, 135.9, 133.1, 132.2, 131.8, 130.3, 128.1, 127.4, 98.6, 58.5. MS m/e 424 (M+).

**2,5-Bis-[4-bromophenyl]-3-methoxy furan.** The methoxyethane prepared above was dissolved in 5 ml PCl₃ and heated at reflux for 3 hr. The excess PCl₃ was removed by distillation. When treated with ice and water, the residue formed a gummy mass. The mixture was extracted with chloroform, and the organic layer was washed with water, dried (Na₂SO₄) and purified by column chromatography over silica gel using hexane: ether (4:1 to 2:1). An off-white solid in 62% yield was obtained; mp 112-113°C [lit. mp 113°C; R.E. Lutz, *J.Am.Chem.Soc.* **51,** 3008 (1929)]. IR (KBr) 3062, 2908, 2877, 1617, 490, 1391, 1211, 1160, 1099, 1073, 1034, 1006, 925, 827, 787. ¹H NMR (CDC₁₃) 7.69 (d, 2H, J=8.8), 7.67.5 (m, 4H), 7.47 (d, 2H, J=8.8), 6.64 (s, 1H), 3.9 (s, 3H). ¹³C NMR(CDCl₃) 149.7, 147.5, 135.5, 131.9, 131.5, 129.4, 129.3, 125.0, 124.5, 121.5, 119.3, 98.6, 58.6. MS m/e 408 (M⁺).

**2,5-Bis(4-cyanophenyl)-3-methoxy furan.** A mixture of 2,5-bis(bromophenyl)-3-methoxyfuran (4.08 g, 0.01 mole) and cuprous cyanide (3.09 g, 0.035 mole) in 10 ml dry N-methyl-2-pyrrolidone was heated ca. 200°C under N₂ for 2.5 hr. The mixture was cooled and poured into 200 ml of water and the precipitated yellow-brown solid was filtered and washed thoroughly with water. The solid was resuspended in water (50 ml) and 100 ml of 10% NaCN and stirred for 2 hr. The slurry was filtered, washed with water, dried and suspended in 250 ml of acetone and passed through a neutral alumina column. On elution with acetone a yellow solid resulted. On recrystallization from CHCl₃:ether (1:1) it gave (1.8 g, 60%) mp 257-258°C. IR (KBr) 3128, 2223, 1608, 1599, 1501, 1409, 1174, 1163, 1027, 924, 836, 815, 651, 537 cm⁻¹. ¹H NMR (DMSO/45°C) 8.03 (d, 2H, J=8.3), 7.95 (d, 2H, J=8.79), 7.91 (d. 2H, J=8.3), 7.85 (d, 2H, J=8.79), 7.62 (s, 1H), 4.0 (s, 3H). ¹³C NMR (DMSO/45°C) 150.0, 149.8, 134.6, 133.4, 133.2, 132.7, 132.5, 124.0, 122.7, 118.9, 118.5, 110.0, 107.6, 102.4, 59.0. MS m/e 300 (M+). Anal. Calculated for: C₁₉H₁₂N₂O (300.31): C, 75.98; H, 4.03; N, 9.33; Found: C, 76.02; H, 4.04; N, 9.36.

**2,5-Bis[4-(2-imidazolinyl)phenyl]-3-methoxyfuran.** The bis-nitrile prepared above (0.9 g, 0.003 mole) was suspended in 70 ml dry ethanol, saturated with dry HCl gas at 0-5°C and stirred under dry conditions for 3-4 days. The mixture was diluted with 200 ml dry ether and the yellow amidate ester was filtered and washed with dry ether and the solid was dried *in vacuo* for 5-6 hr to yield 1.2 g (86%). The solid was resuspended in 30 ml dry ethanol and refluxed gently with 0.46 g (0.008 mole) dry ethylenediamine for 12 hr. The solvent was removed by distillation. The residue was suspended with 50 ml cold water and made basic with 1 M NaOH. The yellow precipitate was filtered, washed with water and dried. Recrystallization from ethanol-ether mixture yielded 0.74 g (75%) mp 186-187°C (dec.). IR (KBr) 3444, 3245, 2931, 2857, 1601, 1512, 1397, 1366, 1277, 1162, 1104, 1031, 926, 842, 743, 670 cm⁻¹. ¹H (DMSO-d₆/60°C) 7.93-7.86 (m, 8H), 7.32 (s, 1H), 3.98 (s, 3H), 3.69 (s, 4H), 3.67 (s, 4H). ¹³C NMR (DMSO-d₆/60°C: 163.3, 163.1, 150.0, 148.6, 138.3, 134.7, 131.9, 131.3, 128.9, 127.6, 126.1, 123.0, 121.9, 100.6, 58.7, 49.0, 48.5. MS m/e 386 (M+).

The free base 0.58 g (0.0015 mole) was dissolved in 10 ml hot ethanol and treated with 10 ml sat. ethanolic HCl. The mixture was heated at reflux for 30 min. The volume was reduced under vacuum to 5-6 ml. The resulting mixture was diluted to 60 ml of dry ether. The yellow crystalline solid obtained was filtered, washed with dry ether and dried *in vacuo* at 60°C for 12 to yield 0.62 g (83%), mp 189-190°C (dec.). IR (KBr): 3422, 3128, 2975, 1599, 1510, 1405, 1363, 1285, 1207, 1028, 845, 666 cm⁻¹. ¹H (D₂O/TSP/50°C) 7.52-7.43 (m, 8H), 6.87 (s, 1H), 3.92 (s, 3H), 3.86 (s, 8H). ¹³C (D₂O/TSP/50°C) 167.2, 153.1, 152.4, 137.6, 137.6, 137.2, 130.9, 130.7, 126.5, 125.4, 122.1, 119.8, 104.2, 61.5, 47.0, 46.9. Anal. Calculated for: C₂₃H₂₂N₄O₂-0.5 H₂O-2HCl: C, 58.97; H, 5.38; N, 11.96. Found: C, 59.16; H, 5.35; N, 11.80.

### EXAMPLE 15

### Preparation of 2,5-Bis[4(N-cyclopropylamidino)phenyl furan

A mixture of the imidate ester (1.3 g, 0.003 mole), cyclopropylamino (0.43 g, 0.0075 mole) in 35 ml of dry ethanol was stirred overnight. The solvent was removed *in vacuo* and water was added to make a yellow solution. The solution was made basic with 1 M NaOH while cooling and stirring The solid which formed was filtered, washed with water and dried. The solid was dissolved in chloroform, dried over Na₂SO₄ and the solvent removed. The residue was recrystallized from ether:CHCl₃ (5:1) to give a pale yellow solid 0.8 g (709%) mp 185-186°C (dec.). IR (KBr): 3464, 3320, 3080, 1610, 1510, 1364, 1022, 848, 791 cm⁻¹. ¹H NMR (CDC₁₃): 7.71 (br s, 8H), 6.78 (s, 2H), 5.3 (v br, 4H), 2.6 (br m, 2H), 0.87-0.81 (m, 4H), 0.67-0.62 (m, 4H). ¹³C NMR (CDC₁₃ + DMSO-d₆): 159.6, 152.2, 134.8, 130.7, 126.4, 122.6, 107.7, 25.7, 6.04. MS m/e 388 (M+).

The free base (0.6 g, 0.0015 mole) was suspended in 3 ml of dry ethanol and was treated with 6 ml ethanolic HCl and heated gently at 65°C for 1 hr. The yellow solution was diluted with 50 ml dry ether and filtered, washed with dry ether and dried *in vacuo* at 75°C for 12 hr. The yield of yellow solid was 0.55 g (80%), mp>310°C (dec.). IR (KBr): 3369, 3181, 3037, 1665, 1607, 1502, 1032, 782, 674 cm⁻¹. ¹H NMR (DMSO-d₆): 10.24 (s, 2H), 9.86 (s, 2H), 9.27 (s, 2H), 8.06 (d, 4H, J=7.94), 7.95 (d, 4H, J=8.54), 7.42 (s, 2H), 2.87 (br m, 2H), 1.09-0.85 (m, 8H). ¹³C NMR (DMSO-d₆): 163.9, 152.3, 133.7, 129.1, 126.6, 123.5, 111.3, 24.7, 6.5. Anal. Calculated for: C₂₄H₂₄N₄O-2HCl: Cal.C, 63.02; H, 5.73; N, 12.25. Found: C, 62.89; H, 5.95; N, 12.00.

### EXAMPLE 16

In **Examples 16-19,** results are presented for a series of compounds. The following compound designations are used throughout.

| Compound | Name |
|---|---|
| 1 | 2,5-bis(4-amidinophenyl)furan |
| 2 | 2,5-bis[4-(2-imidazolinyl)phenyl]furan |
| 3 | 2,5-di-p[2(3,4,5,6-tetrahydropyrimidyl)phenyl]furan |
| 4 | 2,5-bis[4-(4,5,6,7-tetrahydro-1H-1,3-diazepin-2-yl)phenyl]furan |
| 5 | 2,5-bis[4-(3a,4,5,6,7,7a-hexahydro-1H-benzimidazol-2-yl)phenyl]furan |
| 6 | 2,5-bis{4[2-(N-2-hydroxyethyl)imidazolinyl]-phenyl}furan |
| 7 | 2,5-bis-{4-[2-(N-ethylimidazolinyl)]phenyl}furan |
| 8 | 2,5-bis(4-amidinophenyl)-3,4-dimethyl furan |
| 9 | 2,5-[bis{4-(2-imidazolinyl)}phenyl]3-p-tolyloxy furan |
| 10 | 2,5-[bis{4-(2-tetrahydropyrimidinyl)}phenyl]3]p]tolyloxy furan |
| 11 | 2,5-bis{4-[5-(N-2-aminoethylamido)benzimidazol-2-yl]phenyl}furan |
| 12 | 2,5-bis(4-N,N-dimethylcarboxhydrazidephenyl) furan |
| 13 | 2,5-bis[4-(N-isopropylamidino)phenyl]furan |
| 14 | 2,5-bis{4-[3-(dimethylaminopropyl)amidino]phenyl}furan, |
| 15 | 2,5-bis-[4-N-(cyclopropylamidino)phenyl]furan |
| 16 | 2,5-bis-{4-[(N-2-hydroxyethyl)amidino]phenyl}furan |
| 17 | 2,5-bis[4-N-(dimethylaminoethyl)amidino]phenyl furan |
| 18 | 2,5-bis[2-(imidazolinyl)phenyl]-3,4-bis(methoxymethyl)furan |
| 19 | 2,5-bis-{4-[N-(3-aminopropyl)amidino]phenyl}furan |
| 20 | 2,5-bis[4-(N-isopropylamidino)phenyl]-3-methylfuran, |

### DNA Thermal Melting

Thermal melting curves for DNA and its complexes with compounds from **Examples 4, 6,** and **7-9** are determined as previously described in F.A. Tanious, et al., *J.Biomol. Structure & Dynamics* **11**:1063 (1994) and W.D. Wilson, et al., *Biochemistry* **32**:4098 (1993), by following the adsorption change at 260 nm as a function of temperature. Tm values were determined from first derivative plots. Compounds are compared by the increase in Tm (ΔTm=Tm of complex - Tm of the free nucleic acid) they produce in MES buffer (0.01M 2-(N-morpholino) ethanesulfonic acid, 0.001 M EDTA, 0.1 M NaCl adjusted to pH 6.0) at saturating amounts of compound (a ratio of 0.3 moles of compound to nucleic acid bases) unless otherwise indicated.

**Table 1**

| Nucleic Acid Binding Results for Dicationic Diarylfurans | | |
|---|---|---|
| Compound | ΔTm | |
| | DNA¹ | Oligomer² |
| 1 | 25 | 11.7 |
| 2 | 24 | 11.4 |
| 3 | >28 | 13.5 |
| 4 | >28 | 10.7 |
| 5 | 24.5 | 5.6 |
| 6 | 12.2 | 1.0 |
| 7 | 11.8 | -- |
| 9 | 18.1 | -- |
| 10 | >28 | -- |
| 11 | -26 | -- |
| 13 | 23.1 | -- |
| 14 | >28 | -- |
| 15 | >28 | -- |
| 16 | 21.1 | -- |

| | | |
|---|---|---|
| ¹Increase in thermal melting of poly A poly T. See, W.D. Wilson, et al., *Biochemistry* **32**:4098 (1993). | | |
| ²Increase in thermal melting of the oligomer d(GCGCAATTGCGC)₂. See, F.A. Tanious, et al., *J. Biomol. Structure & Dynamics* **11**:1063 (1994). | | |

### EXAMPLE 17

### Topoisomerase II Inhibition and Giardia lamblia Inhibition by Dicationic Diarylfurans

**Table 2**

| Topoisomerase II inhibition and Anti-*Giardia lamblia* | | | | |
|---|---|---|---|---|
| Compound | IC₅₀(µM) | | | |
| | Topoisomerase Activity | | | *Giardia lamblia*⁴ |
| | II | | I³ | |
| | *G.l.*¹ | *D.m.*² | | |
| 1 | 0.5 - 1 | 2.0 | 100 | 0.2 |
| 2 | 3 - 5 | 8 | 50 - 100 | 0.8 |
| 3 | 0.5 - 1 | 2.5 | > 100 | 0.06 |
| 4 | 1.5 | 25 - 50 | -- | 1.1 |
| 5 | 3 - 6 | 50 | -- | 1.7 |
| 6 | 20 | 200 | -- | 5.3 |
| 7 | 25 | -- | -- | -- |
| 8 | 0.75 | 2.4 | <3.12 | 0.26 |
| 9 | 2 - 4 | 25 | 12.5 | >100 |
| 10 | 1 - 3 | 50 - 100 | -- | 3.3 |
| 11 | 5.0 | >300 | -- | 0.62 |
| 12 | 0.6 - 1.2 | 30 - 60 | -- | 0.35 |
| 13 | 0.5 - 1 | 15 - 30 | -- | 0.14 |
| 14 | 0.25 | >6.2 | -- | 8.98 |
| 15 | 1.25 | -- | -- | 0.63 |
| 16 | 1.2 - 2.5 | 25 - 50 | -- | 0.78 |
| 17 | 0.8 | 12.5 | -- | 10.78 |
| 18 | 0.8 - 1 | 12.5 | -- | 4.42 |
| 19 | 0.625 | 25 | -- | 0.059 |
| 20 | 1 - 2 | 3 | 50 - 100 | 4.4 |

| | | | | |
|---|---|---|---|---|
| ¹50% inhibition of topoisomerase II isolated from *Giardia lamblia*. See, C.A. Bell, et al., *Antimicrob. Agents and Chemother*. **37**:2668 (1993). | | | | |
| ²50% inhibition of topoisomerase II isolated from *Drosophila melanogaster*. | | | | |
| ³50% inhibition of topoisomerase I isolated from *Pneumocystis carinii.* | | | | |
| ⁴50% inhibition of growth of *Giardia lamblia* in *in vitro* culture. See, C.A. Bell, et al., *supra.* | | | | |

### EXAMPLE 18

### Activity Against Pneumocystis Carinii pneumonia

**Table 3**

| *In vivo* Activity of Dicationic Diaryl Furans Against *Pneumocystis carinii* | | | |
|---|---|---|---|
| Compound | Dosage¹ (µM/kg/day) | Toxicity² | cyst/g lung³ (% of control) |
| Saline | | 0 | 100 |
| Pentamidine | 22.1 | +2 | 3.66 |
| 1 | 13.3 | 0 | 2.1 |
| | 2.7 | 0 | 8.3 |
| | 0.27 | 0 | 4.8 |
| | 0.027 | 0 | 55.9 |
| | 66.3⁴ | 0 | 28.4 |
| 2 | 23.3 | +2 | 35.4 |
| 3 | 10.9 | +2 | 0.4 |
| 4 | 2.3 | +3 | 2.9 |
| | 0.18 | 0 | 115.4 |
| 5 | 9.2 | +1 | 80.5 |
| 6 | 4.8 | +3 | 107.3 |
| 8 | 24.7 | +1 | 0.8 |
| 9 | 9.3 | +2 | 139.5. |
| 10 | 1.8 | +3 | 139.5 |
| 11 | 4.1 | +4 | 27.4 |
| 12 | 7.78 | +3 | 20.7 |
| 13 | 10.8 | 0 | 0.2 |
| 14 | 3.9 | +3 | 107.3 |
| 16 | 10.4 | 0 | 0.6 |

| | | | |
|---|---|---|---|
| ¹Dosage intravenous except as noted. | | | |
| ²A detailed explanation of the toxicity scale is described in R.R. Tidwell, et al., *Antimicrob. Agents and Chemother*. **37**:1713 (1993). Generally the larger the value the more severe the toxicity. Values greater than 2 indicate deaths of some animals. | | | |
| ³Counted cysts in a blinded protocol in lung tissue reported as percentage of saline-treated controls. See, R.R. Tidwell, et al., *supra.* | | | |
| ⁴Oral dosage by gavage. | | | |

### EXAMPLE 19

### Activity Against Cryptosporidium parvum

**Table 4**

| Activity of Dicationic Diaryl Furans Against *Cryptosporidium parvum* | | | | | | |
|---|---|---|---|---|---|---|
| Compound | No. of mice¹ | Oocysts² | Std Error³ | % Reduction | Score⁴ | Prob.⁵ |
| control | 28 | 36.71 | 2.99 | - | - | - |
| 1 | 21 | 6.6 | 1.9 | 66 | 2.96 | .0015 |
| 13 | 23 | 2.13 | 0.37 | 94.2 | 6.07 | <.0001 |

| | | | | | | |
|---|---|---|---|---|---|---|
| ¹Suckling ICR outbred Swiss mice. *See,* Blagburn et al., *Antimicrobial Agents and Chemotherapy* **35:**1520 (1991). | | | | | | |
| ²Mean oocyst count. | | | | | | |
| ³Standard error of the mean. | | | | | | |
| ⁴Mann-Whitney Z Score. | | | | | | |
| ⁵Probability. | | | | | | |

### EXAMPLE 20

### Preparation of 2,5-Bis[4-(N-2-methoxyethylamidino)phenyl] furan

To a stirred suspension of 0.87 g (0.002 mole) of the furan bis-imidate ester hydrochloride in 15 ml absolute ethanol is added 0.45 g (0.006 mole) freshly distilled 2-methoxy-ethylamine. The mixture is stirred for 12 hours, the solvent is distilled under vacuum, ice-water is added, and the mixture is basified with 1 M NaOH to pH 10. An oily paste separates from the water, it is washed with water, dissolved in CHCl₃ and dried over anhydrous sodium sulfate. The solvent is removed under vacuum, and the solid obtained is recrystallized from ether:chloroform(8:1) to give a solid 0.58 g (69%) having a mp of 118-120°C. IR (KBr): 3430, 3370, 2887, 1647, 1603, 1547, 1367, 1192, 1110, 1021, 847, 787, 681 cm⁻¹. ¹H NMR (CDCl₃) δ 7.75 (d, 4H, J=8.5), 7.63 (d, 4H, J=8.5), 6.8 (s, 2H), 3.68-3.61 (m, 4H), 3.60-3.75 (m, 4H), 3.41 (s, 3H). ¹³C NMR (CDCl₃) δ 136.2, 153.0, 136.5, 131.9, 126.6, 123.8, 100.5, 71.4, 58.8, 58.7. MS: m/e 420(M+).

The freebase (0.42 g, 0.001 mole) is dissolved in 4 ml ethanolic HCl and stirred at 40-45°C for 2 hours, the solvent is removed under vacuum and the solid obtained is triturated with dry ether. The solid is filtered, washed with ether and dried in vacuum at 60°C for 12 hours. The yield is 0.44 g (89 %) of yellow solid having a mp of 208-210°C dec. IR (KBr): 3410, 3320, 3095, 1674, 1615, 1503, 1291, 1197, 1112, 788 cm⁻¹. ¹H NMR (DMSO-d6) δ 10.0 (brs, 2H), 9.65 (brs, 2H), 9.24 (brs, 2H), 8.09 (d, 4H, J=8.3), 7.91 (d, 4H, J=8.7), 7.42 (s, 2H), 3.7-3.62 (m, 8H), 3.33 (s, 6H). ¹³C NMR (DMSO-d₆) δ 162.4, 152.4, 133.8, 129.1, 127.3, 123.6, 111.4, 69.0, 58.2, 42.6. Analysis calculated for C₂₄H₂₈N₄O₃•2HCl•1.5H₂O; theory C:55.38, H:6.39, N:10.76; found: C:55.23, H:6.41, N:10.61.

### EXAMPLE 21

### Preparation of 2,5-Bis[4{N-(3-pentylamidino)phenyl}]furan

Freshly distilled 3-aminopentane (0.34 g, 0.004 mole) is added to a stirred suspension of the furan bis-imidate ester (0.65 g, 0.0015 mole) in 10 ml absolute ethanol, after 5 min. the reaction mixture became clear. This mixture is stirred for 12 hours, and the solvent is removed under vacuum. The residue is treated with 10 ml ice-cold water, and basified to pH 10 with 1M NaOH. The off-white precipitate is filtered, washed with water, dried and recrystallized from CHCl₃:ether (1:3) to give 0.51 g (76%) pale solid, having a mp of 155-156°C. IR (KBr): 3245, 3120, 2962, 1593, 1544, 1380, 1194, 848, 784 cm⁻¹. ¹H NMR (DMSO-d₆) δ 157.3, 152.5, 136.7, 130.5, 127.0, 122.8, 108.8, 55.0, 27.3, 10.5. MS: m/e 444(M+).

The freebase (0.35 g, 0.00078 mole) is dissolved in 5 ml of warm dry ethanol. Ethanolic HCl (5 ml) is added, and the solution is allowed to stir at room temperature for 4 hours. The solvent is removed under vacuum and the oil obtained is triturated with dry ether. A yellow solid is collected by filtration, washed with ether and dried at 75°C for 12 hours under vacuum. The yield is 0.36 g (90%) of product having a mp>360°. IR (KBr): 3410, 3235, 3105, 1668, 1613, 1500, 1459, 1368, 1126, 1025 cm⁻¹. ¹H NMR (D₂O/DMSO-d₆/45°C) δ 7.93 (d, 4H, J=8.5), 7.76 (d, 4H, J=8.5), 7.13 (s,2H), 3.88-3.65 (m, 2H), 1.9-1.8 (m, 4H), 1.78-1.66 (m, 4H), 1.05 (t, 6H, J=7.3). ¹³C NMR(D₂O/DMSOd6/45°C) δ 165.1, 154.1, 136.2, 130.0, 129.0, 125.8, 112.9, 59.1, 27.8, 11.5. Analysis calculated for C₂₈H₃₆N₄O•2HCl•1.5H2O; theory: C:58.32, H:7.16, N:9.71; found: C:58.26, H:7.31, N:9.63.

### EXAMPLE 22

### Preparation of 2,5-Bis[4-cyanophenyl]furan

To a suspension of NaH (2.5 g, 0.11 mole) in 50 ml dry THF under nitrogen is added a solution of diethyl carbonate (11.8 g, 0.1 mole) in 20 ml dry THF. After stirring for 5 min. 4'-bromoacetophenone (19/9 g, 0.1 mole) in 50-60 ml dry THF is added dropwise over 3-4 hours. The yellow reaction mixture is stirred overnight, the solvent is removed under vacuum, and the remaining oil is diluted with water. The mixture is then extracted with 2x100 ml portions of ether. The ether is dried over anhydrous sodium sulfate, and removed to yield a pale oil. The oil is purified by column chromatography over silica gel (elution: hexane-5:1 hexane:ether) or distilled under high vacuum (pressure 0.01 mm) to yield a pale oil 18.8 g (70%). Product is stored at 0°C and used immediately.

Ethyl 3-[4-Bromophenyl]-3-oxopropionate (13.5 g, 0.05 mole), obtained above, in 20 ml dry EtOH is added to a solution of sodium ethoxide (1.15 g Na, 0.05 mole in 30 ml ethanol) under nitrogen, The solution is stirred for 30 min., cooled and 4-bromophenacyl bromide (13.85 g, 0.05 mole) in 75 ml dry EtOH is added slowly over a period of 30-40 min. The mixture is allowed to stir at room temperature for 3 days. The solvent is removed in vacuum, the oil is diluted with water, extracted with ether, washed with water and dried over magnesium sulfate. The solution is filtered and the solvent removed to yield crude oil 16.0 g (68%). Any remaining solvent is removed by placing in vacuum for 2 hours at room temperature.

The crude oil (0.034 mole), ethyl 2,3-bis[4-bromobenzoyl]propionate, is dissolved in 75-80 ml dry EtOH, saturated with dry HCl at 0°C, and allowed to stir at room temperature for 24 hours. The resulting solid is filtered, washed with cold ethanol, and then suspended in water and extracted with CH₂Cl₂. The organic layer is dried over magnesium sulfate and the solvent removed to yield 7.4 g (48%) of the furan ester as a white crystalline solid having a mp of 125-127°C. ¹H NMR(CDCL₃) δ 7.97 (d, 2H, J=8.8), 7.58 (d, 2H, J=8.8), 7.56-7.52 (m, 4H), 7.07 (s, 1H), 4.3 (q, 2H, J=8.8), 1.38 (t, 3H, J=8.8). ¹³C NMR (CDCl₃) δ 163.2, 155.5, 151.5, 132.1, 131.5, 139.8, 128.5, 128.4, 125.5, 123.9, 122.2, 116.4, 108.7, 60.9, 14.3.

This ester (7.0 g, 0.015 mole) is suspended in 75 ml 20% KOH and 10 ml EtOH, and heated under reflux for 4-5 hours. After cooling and acidification with concentrated HCl the solid precipitate is filtered, washed with water, dried in air and in vacuum to yield 5.4 g (82%) of the acid having a mp of 252-254°C. ¹H NMR (CDCl₃) δ 8.03 (d, 2H, J=8.8), 7.77 (d, 2H, J-8.4), 7.67 (d, 2H, J=8.8), 7.62 (d, 2H, J-8.4), 7.37 (s, 1H), 3.4 (br, 1H). ¹³C NMR (CDCl₃) δ 163.8, 153.9, 150.8, 131.7, 131.1, 129.6, 128.2, 128.1, 125.7, 122.7, 121.2, 117.2, 109.6.

A mixture of 2,5-bis(4-bromophenyl)-3-furan carboxylic acid 0.85 g (0.002 mole), CuCN 0.45 g (0.005 mole) in 10 ml freshly distilled quinoline is heated under reflux for 3 hours. The mixture is cooled and 100 ml dilute aqueous HCl is added and the mixture is stirred for 30 min. and filtered. The solid is washed with water and then with hexane. The resultant yellow solid is dissolved in acetone and passed through an alumina (neutral) column to yield a yellow crystalline solid 0.37 g (68%) having a mp of 293-295°C. The product is identical with that described in **Example 1.**

### EXAMPLE 23

### Preparation of 2,5-Bis-(N-isopropylamidino)phenyl]-3-methylfuran

To a suspension of imidate ester dihydrochloride (0.45 g, 0.001 mole) in 10 ml of dry ethanol is added distilled isopropyl amine (0.18 g, 0.003 mole) and the mixture is stirred for 12 hours. The solvent is removed under vacuum and the residue is stirred with 10 ml ice/water, basified with 1 M NaOH and the pH is adjusted to 10. The solid obtained is filtered, dried and crystallized from ether:CHCl₃ (3:1) to yeild 0.31 g (77%) yellow gummy solid. MS: m/e 402(M⁺).

The freebase (0.20 g, 0.0005 mole) is dissolved in 5 ml ethanol and stirred with 5 ml ethanolic HCl for 2 hours. The ethanol is distilled under vacuum and the residue is triturated with dry ether to yield 0.29 g of yellow solid (73%) having a mp of 260-262°C. IR(KBr): 3377, 3210, 3050, 1668, 1611, 1508, 1391, 1128, 932, 842 cm⁻¹. ¹H NMR(D₂O/DMSOd₆) δ 9.65 (br, 2H), 9.55 (br, 2H), 9.7 (br, 2H), 8.0 (d, 2h, J=8.8), 7.94 (d, 2H, J=8.79), 7.91 (d, 2H, J=8.3), 7.84 (d, 2H, J=8.3), 7.29 (s, 1H), 4.10 (brm, 2H), 2.38 (s, 3H), 1.27 (bd, 12H). ¹³C NMR(D₂O/DMSOd₆/65°C) δ 163.1, 163.0, 151.6, 148.2, 136.6, 135.5, 129.3, 129.1, 127.7, 126.9, 125.9, 125.1, 124.3, 115.9, 47.1, 21.9, 13.2. Analysis calculated for C₂₅H₃₀N₄O•2HCl•H₂O: theory: C:60.84, H:6.94, N:11.35; found C:60.79, H:7.01, N:11.27.

The foregoing is illustrative of the present invention and is not to be construed as limiting thereof. The invention is defined by the following claims, with equivalents of the claims to be included therein.

## Claims

1. Use of a compound according to Formula (I): 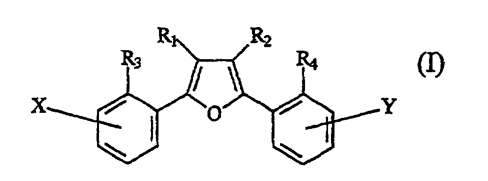 or a pharmaceutically acceptable salt thereof in the manufacture of a medicament for the treatment of *Pneumocystis carinii* pneumonia in a subject in need of such treatment, by administering to said subject an effective *Pneumocystis carinii* -combatting amount of the compound according to Formula (I)
wherein:
R₁ and R₂ are each independently selected from the group consisting of H, C₁₋₆ alkyl, amino C₁₋₆ alkyl, halogen, and oxy C₁₋₆ alkyl;
R₃ and R₄ are each independently selected from the group consisting of H, C₁₋₆ alkyl, oxy C₁₋₆ alkyl, amino C₁₋₆ alkyl and halogen; and
X and Y are located in the *para* positions and are 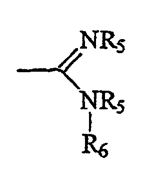 wherein:
each R₅ is independently selected from the group consisting of H, C₁₋₆ alkyl, C₁₋₆ alkoxy C₁₋₆ alkyl, hydroxy C₁₋₆ alkyl, amino C₁₋₆ alkyl, C₁₋₆ alkylamino C₁₋₆ alkyl, and cyclo C₁₋₆ alkyl, or two R₅ groups together represent C₂-C₁₀ alkyl, hydroxy C₁₋₆ alkyl, or C₁₋₆ alkylene; and
R₆ is H, hydroxy, C₁₋₆ alkyl, C₁₋₆ alkoxy C₁₋₆ alkyl, hydroxy C₁₋₆ alkyl, amino C₁₋₆ alkyl, C₁₋₆ alkylamino, C₁₋₆ alkylamino C₁₋₆ alkyl, cyclo C₁₋₆ alkyl, hydroxycyclo C₁₋₆ alkyl, or C₁₋₆ alkoxycycld C₁₋₆ alkyl;
or wherein the compound of Formula I is selected from the group consisting of 2,5-[bis{4-(2-tetrahydropyrimidinyl)}phenyl]3-p(tolyloxy) furan, 2,5-[bis{4-(2-imidazolinyl)}phenyl]3-p(tolyloxy) furan, 2,5-bis[4-(3a,4,5,6,7,7a-hexahydro-1H-benzimidazol-2-yl)phenyl] furan, and 2,5-bis{4-[5-(N-2-aminoethylamido)benzimidazol-2-yl]phenyl} furan;
in an amount effective to treat *Pneumocystis carinii* pneumonia.

2. A use according to Claim 1, wherein said subject is afflicted with *Pneumocystis carinii* pneumonia.

3. A use according to Claim 1, wherein said subject is at risk of developing *Pneumocystis carinii* pneumonia and said compound is administered.in a prophylactically effective amount.

4. A use according to Claim 1, wherein said compound of Formula (I) is selected from the group consisting of compounds wherein
(a) each R₁ is H, R₂ is H, R₃ is H, R₄ is H, R₅ is H, and R₆ is H;
(b) R₁ is H, R₂ is H, R₃ is H, R₄ is H, two R₅ groups together represent C₂ alkyl, and R₆ is H;
(c) R₁ is H, R₂ is H, R₃ is H, R₄ is H, two R₅ groups together represent C₃ alkyl, and R₆ is H;
(d) R₁ is C₁₋₆ alkyl, R₂ is C₁₋₆ alkyl, R₃ is H, R₄ is H, R₅ is H, and R₆ is H;
(e) R₁ is oxy C₁₋₆ alkyl, R₂ is oxy C₁₋₆ alkyl, R₃ is H, R₄ is H, two R₅ groups together represent C₂ alkyl, and R₆ is H;
(f) R₁ is H, R₂ is oxy C₁₋₆ alkyl, R₃ is H, R₄ is H, two R₅ groups together represent C₂ alkyl, and R₆ is H;
(g) R₁ is H, R₂ is tolyloxy C₁₋₆ alkyl, R₃ is H, R₄ is H, two R₅ groups together represent C₂ alkyl, and R₆ is H;
(h) R₁ is H, R₂ is H, R₃ is H, R₄ is H, two R₅ groups together represent C₂ alkyl, and R₆ is hydroxy C₁₋₆ alkyl;
(i) R₁ is H, R₂ is tolyloxy C₁₋₆ alkyl, R₃ is H, R₄ is H, two Rs groups together represent C₃ alkyl, and R₆ is H;
(j) R₁ is H, R₂ is H, R₃ is H, R₄ is H, R₅ is H, and R₆ is amino C₁₋₆ alkyl;
(k) R₁ is H, R₂ is H, R₃ is H, R₄ is H, two groups R₅ groups together represent C₄ alkyl, and R₆ is H;
(l) R₁ is H, R₂ is H, R₃ is H, R₄ is H, R₅ is H, and R₆ is C₁₋₆ alkyl;
(m) R₁ is H, R₂ is H, R₃ is H, R₄ is H, R₅ is H, and R₆ is C₁₋₆ alkylamino;
(n) R₁ is H, R₂ is H, R₃ is H, R₄ is H, R₅ is H, and R₆ is hydroxy C₁₋₆ alkyl;
(o) R₁ is H, R₂ is H, R₃ is H, R₄ is H, R₅ is H, and R₆ is C₁₋₆ alkylamino C₁₋₆ alkyl;
(p) R₁ is H, R₂ is C₁₋₆ alkyl, R₃ is H, R₄ is H, R₅ is H, and R₆ is C₁₋₆ alkyl; and
(q) R₁ is H, R₂ is H, R₃ is H, R₄ is H, two R₅ groups together represent C₂ alkyl, and R₆ is C₁₋₆ alkyl.

5. A use according to Claim 1, wherein said compound of Formula (I) is selected from the group consisting of
2,5-bis(4-amidinophenyl) furan,
2,5-bis(4-amidinophenyl)-3,4-dimethyl furan,
2,5-di-p[2(3,4,5,6-tetrahydropyrimidyl)phenyl] furan,
2,5-bis[4-(2-imidazolinyl)phenyl] furan,
2,5-[bis{4-(2-tetrahydropyrimidinyl)}phenyl]3-p(tolyloxy) furan,
2,5-[bis(4-(2-imidazolinyl)}phenyl]3-p(tolyloxy) furan,
2,5-bis{4[2-(N-2-hydroxyethyl)imidazolinyl]-phenyl} furan,
2,5-bis[4-(N-isopropylamidino)phenyl] furan,
2,5-bis{4-[3-(dimethylaminopropyl)amidino]phenyl} furan,
2,5-bis-{4-[N-(3-aminopropyl)amidino]phenyl} furan,
2,5-bis[2-(imidazolinyl)phenyl]-3,4-bis(methoxymethyl) furan,
2,5-bis[4-N-(dimethylaminoethyl)amidino]phenyl furan,
2,5-bis-{4-[(N-2-hydroxyethyl)amidino]phenyl} furan,
2,5-bis-[4-N-(cyclopropylamidino)phenyl] furan,
2,5-bis-[4-(N,N-diethylaminopropyl)amidino]phenyl furan,
2,5-bis-{4-[2-(N-ethylimidazolinyl)]phenyl} furan,
2,5-bis-{4-[N-(3-pentylamidino)amidino]}phenyl furan,
2,5-bis-[4-(2-imidazolinyl)phenyl]-3-methoxy furan,
2,5-bis[4-(N-isopropylamidino)phenyl]-3-methyl furan,
and the pharmaceutically acceptable salts thereof.

6. Use of a compound according to Formula (I) as defined in claim 1 in the manufacture of a medicament for the treatment of *Giardia lamblia* in a subject in need of such treatment, by administering to said subject a *Giardia lamblia*-combatting amount of the compound according to Formula (I).

7. A use according to Claim 7, wherein said subject is afflicted with *Giardia lamblia*.

8. A use according to Claim 7, wherein said subject is at risk of developing *Giardia lamblia* and said compound is administered in a prophylactically effective amount.

9. A use according to Claim 6, wherein said compound of Formula (I) is selected from the group consisting of compounds wherein
(a) each R₁ is H, R₂ is H, R₃ is H, R₄ is H, R₅ is H, and R₆ is H;
(b) R₁ is H, R₂ is H, R₃ is H, R₄ is H, two R₅ groups together represent C₂ alkyl, and R₆ is H;
(c) R₁ is H, R₂ is H, R₃ is H, R₄ is H, two R₅ groups together represent C₃ alkyl, and R₆ is H;
(d) R₁ is C₁₋₆ alkyl, R₂ is C₁₋₆ alkyl, R₃ is H, R₄ is H, R₅ is H, and R₆ is H;
(e) R₁ is oxy C₁₋₆ alkyl, R₂ is oxy C₁₋₆ alkyl, R₃ is H, R₄ is H, two R₅ groups together represent C₂ alkyl, and R₆ is H;
(f) R₁ is H, R₂ is oxy C₁₋₆ alkyl, R₃ is H, R₄ is H, two R₅ groups together represent C₂ alkyl, and R₆ is H;
(g) R₁ is H, R₂ is tolyloxy C₁₋₆ alkyl, R₃ is H, R₄ is H, two R₅ groups together represent C₂ alkyl, and R₆ is H;
(h) R₁ is H, R₂ is H, R₃ is H, R₄ is H, two R₅ groups together represent C₂ alkyl, and R₆ is hydroxy C₁₋₆ alkyl;
(i) R₁ is H, R₂ is tolyloxy C₁₋₆ alkyl, R₃ is H, R₄ is H, two R₅ groups together represent C₃ alkyl, and R₆ is H;
(j) R₁ is H, R₂ is H, R₃ is H, R₄ is H, R₅ is H, and R₆ is amino C₁₋₆ alkyl;
(k) R₁ is H, R₂ is H, R₃ is H, R₄ is H, two groups R₅ groups together represent C₄ alkyl, and R₆ is H;
(l) R₁ is H, R₂ is H, R₃ is H, R₄ is H, R₅ is H, and R₆ is C₁₋₆ alkyl;
(m) R₁ is H, R₂ is H, R₃ is H, R₄ is H, R₅ is H, and R₆ is C₁₋₆ alkylamino;
(n) R₁ is H, R₂ is H, R₃ is H, R₄ is H, R₅ is H, and R₆ is hydroxy C₁₋₆ alkyl;
(o) R₁ is H, R₂ is H, R₃ is H, R₄ is H, R₅ is H, and R₆ is C₁₋₆ alkylamino C₁₋₆ alkyl;
(p) R₁ is H, R₂ is C₁₋₆ alkyl, R₃ is H, R₄ is H, R₅ is H, and R₆ is C₁₋₆ alkyl; and
(q) R₁ is H, R₂ is H, R₃ is H, R₄ is H, two R₅ groups together represent C₂ alkyl, and R₆ is C₁₋₆ alkyl.

10. A use according to Claim 6, wherein said compound of Formula (I) is selected from the group consisting of
2,5-bis(4-amidinophenyl) furan,
2,5-bis(4-amidinophenyl)-3,4-dimethyl furan,
2,5-di-p[2(3,4,5,6-tetrahydropyrimidyl)phenyl] furan,
2,5-bis[4-(2-imidazolinyl)phenyl] furan,
2,5-[bis{4-(2-tetrahydropyrimidinyl)}phenyl]3-p(tolyloxy) furan,
2,5-[bis{4-(2-imidazolinyl)}phenyl]3-p(tolyloxy) furan,
2,5-bis{4[2-(N-2-hydroxyethyl)imidazolinyl]-phenyl} furan,
2,5-bis[4-(N-isopropylamidino)phenyl] furan,
2,5-bis{4-[3-(dimethylaminopropyl)amidino]phenyl} furan,
2,5-bis-{4-[N-(3-aminopropyl)amidino]phenyl} furan,
2,5-bis[2-(imidazolinyl)phenyl]-3,4-bis(methoxymethyl) furan,
2,5-bis[4-N-(dimethylaminoethyl)amidinophenyl furan,
2,5-bis-{4-[(N-2-hydroxyethyl)amidino]phenyl} furan,
2,5-bis-[4-N-(cyclopropylamidino)phenyl] furan,
2,5-bis-[4-(N,N-diethylaminopropyl)amidino]phenyl furan,
2,5-bis-{4-[2-(N-ethylimidazolinyl)]phenyl} furan,
2,5-bis-{4-[N-(3-pentylamidino) amidino]}phenyl furan,
2,5-bis- [4-(2-imidazolinyl)phenyl]-3-methoxy furan,
2,5-bis[4-(N-isopropylamidino)phenyl]-3-methyl furan,
and the pharmaceutically acceptable salts thereof.

11. Use of a compound according to Formula (I) as defined in claim 1 above in the manufacture of a medicament for the treatment of *Cryptosporidium parvum* in a subject in need of such treatment, by administering to said subject a *Cryptosporidium parvum*-combatting amount of the compound according to Formula (I).

12. A use according to Claim 11, wherein said subject is afflicted with *Cryptosporidium parvum*.

13. A use according to Claim 11, wherein said subject is at risk of developing *Cryptosporidium parvum* and said compound is administered in a prophylactically effective amount.

14. A use according to Claim 11, wherein said compound of Formula (I) is selected from the group consisting of compounds wherein
(a) each R₁ is H, R₂ is H, R₃ is H, R₄ is H, R₅ is H, and R₆ is H;
(b) R₁ is H, R₂ is H, R₃ is H, R₄ is H, two R₅ groups together represent C₂ alkyl, and R₆ is H;
(c) R₁ is H, R₂ is H, R₃ is H, R₄ is H, two R₅ groups together represent C₃ alkyl, and R₆ is H;
(d) R₁ is C₁₋₆ alkyl, R₂ is C₁₋₆ alkyl, R₃ is H, R₄ is H, R₅ is H, and R₆ is H;
(e) R₁ is oxy C₁₋₆ alkyl, R₂ is oxy C₁₋₆ alkyl, R₃ is H, R₄ is H, two R₅ groups together represent C₂ alkyl, and R₆ is H;
(f) R₁ is H, R₂ is oxy C₁₋₆ alkyl, R₃ is H, R₄ is H, two R₅ groups together represent C₂ alkyl, and R₆ is H;
(g) R₁ is H, R₂ is tolyloxy C₁₋₆ alkyl, R₃ is H, R₄ is H, two R₅ groups together represent C₂ alkyl, and R₆ is H;
(h) R₁ is H, R₂ is H, R₃ is H, R₄ is H, two R₅ groups together represent C₂ alkyl, and R₆ is hydroxy C₁₋₆ alkyl;
(i) R₁ is H, R₂ is tolyloxy C₁₋₆ alkyl, R₃ is H, R₄ is H, two R₅ groups together represent C₃ alkyl, and R₆ is H;
(j) R₁ is H, R₂ is H, R₃ is H, R₄ is H, R₅ is H, and R₆ is amino C₁₋₆ alkyl;
(k) R₁ is H, R₂ is H, R₃ is H, R₄ is H, two groups R₅ groups together represent C₄ alkyl, and R₆ is H;
(l) R₁ is H, R₂ is H, R₃ is H, R₄ is H, R₅ is H, and R₆ is C₁₋₆ alkyl;
(m) R₁ is H, R₂ is H, R₃ is H, R₄ is H, R₅ is H, and R₆ is C₁₋₆ alkylamino;
(n) R₁ is H, R₂ is H, R₃ is H, R₄ is H, R₅ is H, and R₆ is hydroxy C₁₋₆ alkyl;
(o) R₁ is H, R₂ is H, R₃ is H, R₄ is H, R₅ is H, and R₆ is C₁₋₆ alkylamino C₁₋₆ alkyl;
(p) R₁ is H, R₂ is C₁₋₆ alkyl, R₃ is H, R₄ is H, R₅ is H, and R₆ is C₁₋₆ alkyl; and
(q) R₁ is H, R₂ is H, R₃ is H, R₄ is H, two R₅ groups together represent C₂ alkyl, and R₆ is C₁₋₆ alkyl.

15. A use according to Claim 11, wherein said compound of Formula (I) is selected from the group consisting of
2,5-bis(4-amidinophenyl) furan,
2,5-bis(4-amidinophenyl)-3,4-dimethyl furan,
2,5-di-p[2(3,4,5,6-tetrahydropyrimidyl)phenyl] furan,
2,5-bis [4-(2-imidazolinyl)phenyl] furan,
2,5-[bis{4-(2-tetrahydropyrimidinyl)}phenyl]3-p(tolyloxy) furan,
2,5-[bis{4-(2-imidazolinyl)}phenyl]3-p(tolyloxy) furan,
2,5-bis{4[2-(N-2-hydroxyethyl)imidazolinyl]-phenyl} furan,
2,5-bis[4-(N-isopropylamidino)phenyl] furan,
2,5-bis{4-[3-(dimethylaminopropyl)amidino]phenyl} furan,
2,5-bis-{4-[N-(3-aminopropyl)amidino]phenyl} furan,
2, 5-bis [2-(imidazolinyl)phenyl]-3,4-bis(methoxymethyl) furan,
2,5-bis[4-N-(dimethylaminoethyl)amidino]phenyl furan,
2,5-bis-{4-[(N-2-hydroxyethyl)amidino]phenyl} furan,
2,5-bis-[4-N-(cyclopropylamidino)phenyl] furan,
2,5-bis-[4-(N,N-diethylaminopropyl)amidino]phenyl furan,
2,5-bis-{4-[2-(N-ethylimidazolinyl)]phenyl} furan,
2,5-bis-{4-[N-(3-pentylamidino)amidino]}phenyl furan,
2,5-bis-[4-(2-imidazolinyl)phenyl]-3-methoxy furan,
2,5-bis[4-(N-isopropylamidino)phenyl]-3-methyl furan,
and the pharmaceutically acceptable salts thereof.

16. A compound according to Formula (I): 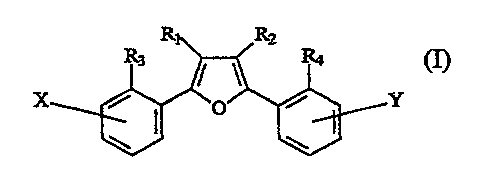 wherein:
X and Y are located in the *para* position and are each 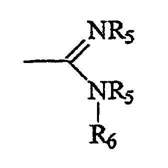 and wherein said compounds of Formula (I) are selected from the group consisting of compounds wherein:
(a) R₁ is H, R₂ is H or C₁₋₆ alkyl, R₃ is H, R₄ is H, R₅ is H, and R₆ is isoalkyl;
(b) R₁ is H, R₂ is H, R₃ is H, R₄ is H, R₅ is H, and R₆ is C₃-C₈ alkoxyalkyl;
(c) R₁ is H, R₂ is H or C₁₋₆ alkyl, R₃ is H, R₄ is H, R₅ is H, and R₆ is ethylhydroxy, propylhydroxy, butylhydroxy, pentylhydroxy, or hexylhydroxy;
(d) R₁ is H, R₂ is H or C₁₋₆ alkyl, R₃ is H, R₄ is H, R₅ is H, and R₆ is propoxyethyl;
(e) R₁ is H, R₂ is H or C₁₋₆ alkyl, R₃ is H, R₄ is H, R₅ is H, and R₆ is propoxyisopropyl;
(f) R₁ is H, R₂ is H or C₁₋₆ alkyl, R₃ is H, R₄ is H, R₅ is H, and R₆ is tolyloxy, benzyl, tolyloxy C₁₋₆ alkyl or benzyl C₁₋₆ alkyl; and
(g) R₁ is H, R₂ is H or C₁₋₆ alkyl, R₃ is H, R₄ is H, R₅ is H, and R₆ is C₁₋₆ alkylcyclo C₁₋₆ alkyl;
and pharmaceutically acceptable salts thereof.

## Patentansprüche

1. Verwendung einer Verbindung nach Formel (I): 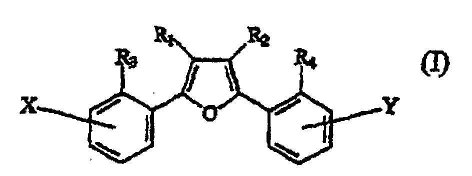 oder ein pharmazeutisch zulässiges Salz davon in der Herstellung eines Medikaments für die Behandlung von *Pneumocystis carinii*-Pneumonie bei einem einer solchen Behandlung bedürftigen Patienten durch Verabreichung einer wirksamen, *Pneumocystis carinii* bekämpfenden Menge der Verbindung nach Formel (I) an den Patienten, in welcher Formel sind:
R₁ und R₂ jeweils unabhängig ausgewählt aus der Gruppe, bestehend aus: H, C₁₋₆-Alkyl, Amino-C₁₋₆-alkyl, Halogen- und Oxy-C₁₋₆-alkyl;
R₃ und R₄ jeweils unabhängig ausgewählt aus der Gruppe, bestehend aus: H, C₁₋₆-Alkyl, Oxy-C₁₋₆-alkyl, Amino-C₁₋₆-alkyl und Halogen-; und
X und Y befinden sich in den para-Stellungen und sind 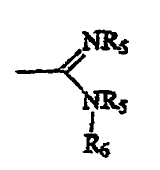 worin sind:
jedes R₅ ausgewählt ist aus der Gruppe, bestehend aus: H, C₁₋₆-Alkyl, C₁₋₆-Alkoxy-C₁₋₆-alkyl, Hydroxy-C₁₋₆-alkyl, Amino-C₁₋₆-alkyl, C₁₋₆-Alkylamino-C₁₋₆-alkyl und Cyclo-C₁₋₆-alkyl, oder zwei R₅-Gruppen stellen gemeinsam C₂-C₁₀-Alkyl, Hydroxy-C₁₋₆-alkyl dar, oder C₁₋₆-Alkylen; und
R₆ ist H, Hydroxy, C₁₋₆-Alkyl, C₁₋₆-Alkoxy-C₁₋₆-alkyl, Hydroxy-C₁₋₆-alkyl, Amino-C₁₋₆-alkyl, C₁₋₆-Alkylamino, C₁₋₆-Alkylamino-C₁₋₆-alkyl, Cyclo-C₁₋₆-alkyl, Hydroxycyclo-C₁₋₆-alkyl, oder C₁₋₆-Alkoxycyclo-C₁₋₆-alkyl;
oder worin die Verbindung der Formel I ausgewählt ist aus der Gruppe, bestehend aus: 2,5-[Bis{4-(2-tetrahydropyrimidinyl)}phenyl]3-p(tolyloxy)-furan, 2,5-[Bis{4-(2-imidazolinyl)}phenyl]3-p(tolyloxy)-furan, 2,5-Bis[4-(3a,4,5,6,7,7a-hexahydro-1H-benzimidazol-2-yl)phenyl]-furan und 2,5-Bis{4-[5-(N-2-aminoethylamido)benzimidazol-2-yl]phenyl}-furan; in einer wirksamen Menge zur Behandlung von *Pneumocystis carinii*-Pneumonie.

2. Verwendung nach Anspruch 1, wobei der Patient erkrankt ist an *Pneumocystis carinii*-Pneumonie.

3. Verwendung nach Anspruch 1, wobei der Patient gefährdet ist, eine *Pneumocystis carinii*-Pneumonie zu entwickeln und die Verbindung in einer prophylaktisch wirksamen Menge verabreicht wird.

4. Verwendung nach Anspruch 1, wobei die Verbindung der Formel (I) ausgewählt ist aus der Gruppe, bestehend aus Verbindungen, bei denen sind:
(a) jedes R₁ ist H, R₂ ist H, R₃ ist H, R₄ ist H, R₅ ist H und R₆ ist H;
(b) R₁ ist H, R₂ ist H, R₃ ist H, R₄ ist H, zwei R₅-Gruppen stellen gemeinsam C₂-Alkyl und R₆ ist H;
(c) R₁ ist H, R₂ ist H, R₃ ist H, R₄ ist H, zwei R₅-Gruppen stellen gemeinsam C₃-Alkyl dar und R₆ ist H;
(d) R₁ ist C₁₋₆-Alkyl, R₂ ist C₁₋₆-Alkyl, R₃ ist H, R₄ ist H, R₅ ist H und R₆ ist H;
(e) R₁ ist Oxy-C₁₋₆-alkyl, R₂ ist Oxy-C₁₋₆-alkyl, R₃ ist H, R₄ ist H, zwei R₅-Gruppen stellen gemeinsam C₂-Alkyl dar und R₆ ist H;
(f) R₁ ist H, R₂ ist Oxy-C₁₋₆-alkyl, R₃ ist H, R₄ ist H, zwei R₅-Gruppen stellen gemeinsam C₂-Alkyl dar und R₆ ist H;
(g) R₁ ist H, R₂ ist Tolyloxy-C₁₋₆-alkyl, R₃ ist H, R₄ ist H, zwei R₅-Gruppen stellen gemeinsam C₂-Alkyl dar und R₆ ist H;
(h) R₁ ist H, R₂ ist H, R₃ ist H, R₄ ist H, zwei R₅-Gruppen stellen gemeinsam C₂-Alkyl dar und R₆ ist Hydroxy-C₁₋₆-alkyl;
(i) R₁ ist H, R₂ ist Tolyloxy-C₁₋₆-alkyl, R₃ ist H, R₄ ist H, zwei R₅-Gruppen stellen gemeinsam C₃-Alkyl dar und R₆ ist H;
(j) R₁ ist H, R₂ ist H, R₃ ist H, R₄ ist H, R₅ ist H und R₆ ist Amino-C₁₋₆-alkyl;
(k) R₁ ist H, R₂ ist H, R₃ ist H, R₄ ist H, zwei R₅-Gruppen stellen gemeinsam C₄-Alkyl dar und R₆ ist H;
(l) R₁ ist H, R₂ ist H, R₃ ist H, R₄ ist H, R₅ ist H und R₆ ist C₁₋₆-Alkyl;
(m) R₁ ist H, R₂ ist H, R₃ ist H, R₄ ist H, R₅ ist H, und R₆ ist C₁₋₆-Alkylamino;
(n) R₁ ist H, R₂ ist H, R₃ ist H, R₄ ist H, R₅ ist H und R₆ ist Hydroxy-C₁₋₆-alkyl;
(o) R₁ ist H, R₂ ist H, R₃ ist H, R₄ ist H, R₅ ist H und R₆ ist C₁₋₆-Alkylamino-C₁₋₆-alkyl;
(p) R₁ ist H, R₂ ist C₁₋₆-Alkyl, R₃ ist H, R₄ ist H, R₅ ist H und R₆ ist C₁₋₆-Alkyl; und
(q) R₁ ist H, R₂ ist H, R₃ ist H, R₄ ist H, zwei R₅-Gruppen stellen gemeinsam C₂-Alkyl dar und R₆ ist C₁₋₆₋Alkyl.

5. Verwendung nach Anspruch 1, wobei die Verbindung nach Formel (I) ausgewählt ist aus der Gruppe, bestehend aus:
2,5-Bis(4-amidinophenyl)-furan,
2,5-Bis(4-amidinophenyl)-3,4-dimethylfuran,
2,5-Di-p[2(3,4,5,6-tetrahydropyrimidyl)phenyl]-furan,
2,5-Bis[4-(2-imidazolinyl)phenyl]-furan,
2,5-[Bis{4-{2-tetrahydropyrimidinyl)}phenyl]3-p(tolyloxy)-furan,
2,5-[Bis{4-(2-imidazolinyl)}phenyl]3-p(tolyloxy) furan,
2,5-Bis {4[2-(N-2-hydroxyethyl)imidazolinyl]-phenyl}-furan,
2,5-Bis[4-(N-isopropylamidino)phenyl]-furan,
2,5-Bis{4-[3-(dimethylaminopropyl)amidino]phenyl} -furan,
2,5-Bis-{4-[N-(3-aminopropyl)amidino]phenyl}-furan,
2,5-Bis[2-(imidazolinyl)phenyl]-3,4-bis(methoxymethyl}-furan,
2,5-Bis[4-N-(dimethylaminoethyl)amidino]phenylfuran,
2,5-Bis-{4-[(N-2-hydroxyethyl)amidino]phenyl}-furan,
2,5-Bis-[4-N-(cyclopropylamidino)phenyl]-furan,
2,5-Bis-[4-(N,N-diethylaminopropyl)amidino]phenylfuran,
2,5-Bis-{4-[2-(N-ethylimidazolinyl)]phenyl}-furan,
2,5-Bis-{4-[N-(3-pentylamidino)amidino]phenylfuran,
2,5-Bis-[4-(2-imidazolinyl)phenyl]-3-methoxyfuran,
2,5-Bis-[4-(N-isopropylamidino)phenyl]-3-methylfuran,
sowie die pharmazeutisch zulässigen Salze davon.

6. Verwendung einer Verbindung der Formel (I) nach Anspruch 1 in der Herstellung eines Medikaments für die Behandlung von *Giardia lamblia* bei einem einer solchen Behandlung bedürftigen Patienten durch Verabreichung einer *Giardia lamblia* bekämpfenden Menge der Verbindung nach Formel (I).

7. Verwendung nach Anspruch 7, wobei der Patient von *Giardia lamblia* befallen ist.

8. Verwendung nach Anspruch 7, wobei der Patient gefährdet ist, *Giardia lamblia zu* entwickeln und die Verbindung in einer prophylaktisch wirksamen Menge verabreicht wird.

9. Verwendung nach Anspruch 6, wobei die Verbindung der Formel (I) ausgewählt ist aus der Gruppe, bestehend aus Verbindungen, bei denen sind:
(a) jedes R₁ ist H, R₂ ist H, R₃ ist H, R₄ ist H, R₅ ist H und R₆ ist H;
(b) R₁ ist H, R₂ ist H, R₃ ist H, R₄ ist H, zwei R₅-Gruppen stellen gemeinsam C₂-Alkyl dar und R₆ ist H;
(c) R₁ ist H, R₂ ist H, R₃ ist H, R₄ ist H, zwei R₅-Gruppen stellen gemeinsam C₃-Alkyl dar und R₆ ist H;
(d) R₁ ist C₁₋₆-Alkyl, R₂ ist C₁₋₆-Alkyl, R₃ ist H, R₄ ist H, R₅ ist H und R₆ ist H;
(e) R₁ ist Oxy-C₁₋₆-alkyl, R₂ ist Oxy-C₁₋₆-alkyl, R₃ ist H, R₄ ist H, zwei R₅-Gruppen stellen gemeinsam C₂-Alkyl dar und R₆ ist H;
(f) R₁ ist H, R₂ ist Oxy-C₁₋₆-alkyl, R₃ ist H, R₄ ist H, zwei R₅-Gruppen stellen gemeinsam C₂-Alkyl dar und R₆ ist H;
(g) R₁ ist H, R₂ ist Tolyloxy-C₁₋₆-alkyl, R₃ ist H, R₄ ist H, zwei R₅-Gruppen stellen gemeinsam C₂-Alkyl dar und R₆ ist H;
(h) R₁ ist H, R₂ ist H, R₃ ist H, R₄ ist H, zwei R₅-Gruppen stellen gemeinsam C₂-Alkyl dar und R₆ ist Hydroxy-C₁₋₆-alkyl;
(i) R₁ ist H, R₂ ist Tolyloxy-C₁₋₆-alkyl, R₃ ist H, R₄ ist H, zwei R₅-Gruppen stellen gemeinsam C₃-Alkyl dar und R₆ ist H;
(j) R₁ ist H, R₂ ist H, R₃ ist H, R₄ ist H, R₅ ist H und R₆ ist Amino-C₁₋₆-alkyl;
(k) R₁ ist H, R₂ ist H, R₃ ist H, R₄ ist H, zwei R₅-Gruppen stellen gemeinsam C₄-Alkyl dar und R₆ ist H;
(l) R₁ ist H, R₂ ist H, R₃ ist H, R₄ ist H, R₅ ist H und R₆ ist C₁₋₆-Alkyl;
(m) R₁ ist H, R₂ ist H, R₃ ist H, R₄ ist H, R₅ ist H, und R₆ ist C₁₋₆-Alkylamino;
(n) R₁ ist H, R₂ ist H, R₃ ist H, R₄ ist H, R₅ ist H und R₆ ist Hydroxy-C₁₋₆-alkyl;
(o) R₁ ist H, R₂ ist H, R₃ ist H, R₄ ist H, R₅ ist H und R₆ ist C₁₋₆-Alkylamino-C₁₋₆-alkyl;
(p) R₁ ist H, R₂ ist C₁₋₆-Alkyl, R₃ ist H, R₄ ist H, R₅ ist H und R₆ ist C₁₋₆-Alkyl; und
(q) R₁ ist H, R₂ ist H, R₃ ist H, R₄ ist H, zwei R₅-Gruppen stellen gemeinsam C₂-Alkyl dar und R₆ ist C₁₋₆Alkyl.

10. Verwendung nach Anspruch 1, wobei die Verbindung nach Formel (I) ausgewählt ist aus der Gruppe, bestehend aus:
2,5-Bis(4-amidinophenyl)-furan,
2,5-Bis(4-amidinophenyl)-3,4-dimethylfuran,
2,5-Di-p[2(3,4,5,6-tetrahydropyrimidyl)phenyl]-furan,
2,5-Bis[4-(2-imidazolinyl)phenyl]-furan,
2,5-[Bis{4-{2-tetrahydropyrimidinyl)}phenyl]3-p(tolyloxy)-furan,
2,5-[Bis{4-(2-imidazolinyl)}phenyl]3-p(tolyloxy) furan,
2,5-Bis{4[2-(N-2-hydroxyethyl)imidazolinyl]-phenyl}-furan,
2,5-Bis[4-(N-isopropylamidino)phenyl]-furan,
2,5-Bis{4-[3-(dimethylaminopropyl)amidino]phenyl}-furan,
2,5-Bis-{4-[N-(3-aminopropyl)amidino]phenyl} -furan,
2,5-Bis[2-(imidazolinyl)phenyl]-3,4-bis(methoxymethyl}-furan,
2,5-Bis[4-N-(dimethylaminoethyl)amidino]phenylfuran,
2,5-Bis-{4-[(N-2-hydroxyethyl)amidino]phenyl}-furan,
2,5-Bis-[4-N-(cyclopropylamidino)phenyl]-furan,
2,5-Bis-[4-(N,N-diethylaminopropyl)amidino]phenylfuran,
2,5-Bis-{4-[2-(N-ethylimidazolinyl)]phenyl}-furan,
2,5-Bis-{4-[N-(3-pentylamidino)amidino]phenylfuran,
2,5-Bis-[4-(2-imidazolinyl)phenyl]-3-methoxyfuran,
2,5-Bis-[4-(N-isopropylamidino)phenyl]-3-methylfuran,
sowie die pharmazeutisch zulässigen Salze davon.

11. Verwendung einer Verbindung der Formel (I) nach Anspruch 1 in der Herstellung eines Medikaments zur Behandlung von *Cryptosporidium parvum* bei einem einer solchen Behandlung bedürftigen Patienten durch Verabreichung einer wirksamen, *Cryptosporidium parvum* bekämpfenden Menge der Verbindung nach Formel (I) an den Patienten.

12. Verwendung nach Anspruch 11, wobei der Patient von *Cryptosporidium parvum* befallen ist.

13. Verwendung nach Anspruch 11, wobei der Patient gefährdet ist, *Cryptosporidium parvum* zu entwickeln und die Verbindung in einer prophylaktisch wirksamen Menge verabreicht wird.

14. Verwendung nach Anspruch 6, wobei die Verbindung der Formel (I) ausgewählt aus der Gruppe, bestehend aus Verbindungen, bei denen sind:
(a) jedes R₁ ist H, R₂ ist H, R₃ ist H, R₄ ist H, R₅ ist H und R₆ ist H;
(b) R₁ ist H, R₂ ist H, R₃ ist H, R₄ ist H, zwei R₅-Gruppen stellen gemeinsam C₂-Alkyl dar und R₆ ist H;
(c) R₁ ist H, R₂ ist H, R₃ ist H, R₄ ist H, zwei R₅-Gruppen stellen gemeinsam C₃-Alkyl dar und R₆ ist H;
(d) R₁ ist C₁₋₆-Alkyl, R₂ ist C₁₋₆-Alkyl, R₃ ist H, R₄ ist H, R₅ ist H und R₆ ist H;
(e) R₁ ist Oxy-C₁₋₆-alkyl, R₂ ist Oxy-C₁₋₆-alkyl, R₃ ist H, R₄ ist H, zwei R₅-Gruppen stellen gemeinsam C₂-Alkyl dar und R₆ ist H;
(f) R₁ ist H, R₂ ist Oxy-C₁₋₆-alkyl, R₃ ist H, R₄ ist H, zwei R₅-Gruppen stellen gemeinsam C₂-Alkyl dar und R₆ ist H;
(g) R₁ ist H, R₂ ist Tolyloxy-C₁₋₆-alkyl, R₃ ist H, R₄ ist H, zwei R₅-Gruppen stellen gemeinsam C₂-Alkyl dar und R₆ ist H;
(h) R₁ ist H, R₂ ist H, R₃ ist H, R₄ ist H, zwei R₅-Gruppen stellen gemeinsam C₂-Alkyl dar und R₆ ist Hydroxy-C₁₋₆-alkyl;
(i) R₁ ist H, R₂ ist Tolyloxy-C₁₋₆-alkyl, R₃ ist H, R₄ ist H, zwei R₅-Gruppen stellen gemeinsam C₃-Alkyl dar und R₆ ist H;
(j) R₁ ist H, R₂ ist H, R₃ ist H, R₄ ist H, R₅ ist H und R₆ ist Amino-C₁₋₆-alkyl;
(k) R₁ ist H, R₂ ist H, R₃ ist H, R₄ ist H, zwei R₅-Gruppen stellen gemeinsam C₄-Alkyl dar und R₆ ist H;
(l) R₁ ist H, R₂ ist H, R₃ ist H, R₄ ist H, R₅ ist H und R₆ ist C₁₋₆-Alkyl;
(m) R₁ ist H, R₂ ist H, R₃ ist H, R₄ ist H, R₅ ist H, und R₆ ist C₁₋₆-Alkylamino;
(n) R₁ ist H, R₂ ist H, R₃ ist H, R₄ ist H, R₅ ist H und R₆ ist Hydroxy-C₁₋₆-alkyl;
(o) R₁ ist H, R₂ ist H, R₃ ist H, R₄ ist H, R₅ ist H und R₆ ist C₁₋₆-Alkylamino-C₁₋₆-alkyl;
(p) R₁ ist H, R₂ ist C₁₋₆-Alkyl, R₃ ist H, R₄ ist H, R₅ ist H und R₆ ist C₁₋₆-Alkyl; und
(q) R₁ ist H, R₂ ist H, R₃ ist H, R₄ ist H, zwei R₅-Gruppen stellen gemeinsam C₂-Alkyl dar und R₆ ist C₁₋₆-Alkyl.

15. Verwendung nach Anspruch 1, wobei die Verbindung nach Formel (I) ausgewählt ist aus der Gruppe, bestehend aus:
2,5-Bis(4-amidinophenyl)-furan,
2,5-Bis(4-amidinophenyl)-3,4-dimethylfuran,
2,5-Di-p[2(3,4,5,6-tetrahydropyrimidyl)phenyl]-furan,
2,5-Bis[4-(2-imidazolinyl)phenyl]-furan,
2,5-[Bis{4-{2-tetrahydropyrimidinyl)}phenyl]3-p(tolyloxy)-furan,
2,5-[Bis{4-(2-imidazolinyl)}phenyl]3-p(tolyloxy) furan,
2,5-Bis{4[2-(N-2-hydroxyethyl)imidazolinyl]-phenyl}-furan,
2,5-Bis[4-(N-isopropylamidino)phenyl]-furan,
2,5-Bis{4-[3-(dimethylaminopropyl)amidino]phenyl}-furan,
2,5-Bis-{4-[N-(3-aminopropyl)amidino]phenyl}-furan,
2,5-Bis[2-(imidazolinyl)phenyl]-3,4-bis(methoxymethyl}-furan,
2,5-Bis[4-N-(dimethylaminoethyl)amidino]phenylfuran,
2,5-Bis-{4-[(N-2-hydroxyethyl)amidino]phenyl}-furan,
2,5-Bis-[4-N-(cyclopropylamidino)phenyl]-furan,
2,5-Bis-[4-(N,N-diethylaminopropyl)amidino]phenylfuran,
2,5-Bis-{4-[2-(N-ethylimidazolinyl)]phenyl }-furan,
2,5-Bis-{4-[N-(3-pentylamidino)amidino]phenylfuran,
2,5-Bis-[4-(2-imidazolinyl)phenyl]-3-methoxyfuran,
2,5-Bis-[4-(N-isopropylamidino)phenyl]-3-methylfuran,
sowie die pharmazeutisch zulässigen Salze davon.

16. Verbindung nach Formel (I) : 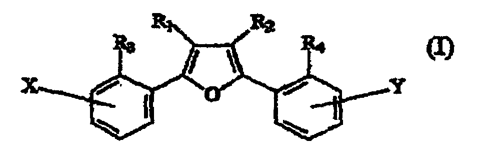 worin sind:
X und Y befinden sich in der *para*-Stellung und sind jeweils: 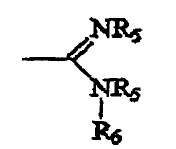 und wobei die Verbindungen der Formula (I) ausgewählt ist aus der Gruppe, bestehend aus Verbindungen, worin sind:
(a) R₁ ist H, R₂ ist H oder C₁₋₆-Alkyl, R₃ ist H, R₄ ist H, R₅ ist H und R₆ ist Isoalkyl;
(b) R₁ ist H, R₂ ist H, R₃ ist H, R₄ ist H, R₅ ist H und R₆ ist C₃-C₈-Alkoxyalkyl;
(c) R₁ ist H, R₂ ist H oder C₁₋₆-Alkyl, R₃ ist H, R₄ ist H, R₅ ist H und R₆ ist Ethylhydroxy, Propylhydroxy, Butylhydroxy, Pentylhydroxy oder Hexylhydroxy;
(d) R₁ ist H, R₂ ist H oder C₁₋₆-Alkyl, R₃ ist H, R₄ ist H, R₅ ist H und R₆ ist Propoxyethyl;
(e) R₁ ist H, R₂ ist H oder C₁₋₆-Alkyl, R₃ ist H, R₄ ist H, R₅ ist H und R₆ ist Propoxyisopropyl;
(f) R₁ ist H, R₂ ist H oder C₁₋₆-Alkyl, R₃ ist H, R₄ ist H, R₅ ist H und R₆ ist Tolyloxy, Benzyl, Tolyloxy-C₁₋₆-alkyl oder Benzyl-C₁₋₆-alkyl und
(g) R₁ ist H, R₂ ist H oder C₁₋₆-Alkyl, R₃ ist H, R₄ ist H, R₅ ist H und R₆ ist C₁₋₆-Alkylcyclo-C₁₋₆-alkyl; sowie pharmazeutisch zulässige Salze davon.

## Revendications

1. Utilisation d'un composé de formule (I): 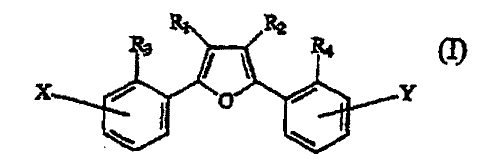 ou d'un de ses sels pharmaceutiquement acceptables dans la fabrication d'un médicament destiné au traitement de la pneumonie à *Pneumocystis carinii* chez un sujet ayant besoin d'un tel traitement, par administration audit sujet d'une quantité efficace pour combattre *Pneumocystis carinii* du composé de formule (I)
dans laquelle:
R₁ et R₂ sont chacun indépendamment choisis dans le groupe constitué de H, d'un groupe alkyle en C₁-C₆, d'un groupe aminoalkyle en C₁-C₆, d'un halogène, et d'un groupe oxyalkyle en C₁-C₆;
R₃ et R₄ sont chacun indépendamment choisis dans le groupe constitué de H, d'un groupe alkyle en C₁-C₆, d'un groupe oxyalkyle en C₁-C₆, d'un groupe aminoalkyle en C₁-C₆ et d'un halogène; et
X et Y sont situés en positions *para* et sont 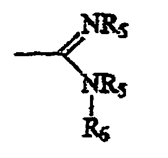 où:
chaque R₅ est indépendamment choisi dans le groupe constitué de H, d'un groupe alkyle en C₁-C₆, d'un groupe (alcoxy en C₁-C₆)alkyle en C₁-C₆, d'un groupe hydroxyalkyle en C₁-C₆, d'un groupe aminoalkyle en C₁-C₆, d'un groupe (alkyl en C₁-C₆)aminoalkyle en C₁-C₆, et d'un groupe cycloalkyle en C₁-C₆, ou deux groupes R₅ ensemble représentent un groupe alkyle en C₂-C₁₀, un groupe hydroxyalkyle en C₁-C₆, ou un groupe alkylène en C₁-C₆; et
R₆ est H, un groupe hydroxy, un groupe alkyle en C₁-C₆, un groupe (alcoxy en C₁-C₆)alkyle en C₁-C₆, un groupe hydroxyalkyle en C₁-C₆, un groupe aminoalkyle en C₁-C₆, un groupe (alkyl en C₁-C₆)amino, un groupe (alkyl en C₁-C₆)aminoalkyle en C₁-C₆, un groupe cycloalkyle en C₁-C₆, un groupe hydroxycycloalkyle en C₁-C₆, ou un groupe (alcoxy en C₁-C₆)cycloalkyle en C₁-C₆;
ou dans laquelle le composé de formule I est choisi dans le groupe constitué du 2,5-[bis{4-(2-tétrahydropyrimidinyl)}phényl]-3-p-(tolyloxy)furane, du 2,5-[bis{4-(2-imidazolinyl)}phényl]-3-p-(tolyloxy)furane, du 2,5-bis[4-(3a,4,5,6,7,7a-hexahydro-1H-benzimidazol-2-yl)phényl]furane, et du 2,5-bis{4-[5-(N-2-aminoéthy l amido)benzimidazol-2-yl]phényl}furane;
en une quantité efficace pour traiter une pneumonie à *Pneumocystis carinii*.

2. Utilisation selon la revendication 1, dans laquelle ledit sujet est atteint d'une pneumonie à *Pneumocystis carinii*.

3. Utilisation selon la revendication 1, dans laquelle ledit sujet est susceptible de développer une pneumonie à *Pneumocystis carinii* et ledit composé est administré en une quantité prophylactiquement efficace.

4. Utilisation selon la revendication 1, dans laquelle ledit composé de formule (I) est choisi dans le groupe constitué des composés dans lesquels:
(a) chaque R₁ est H, R₂ est H, R₃ est H, R₄ est H, R₅ est H, et R₆ est H;
(b) R₁ est H, R₂ est H, R₃ est H, R₄ est H, deux groupes R₅ ensemble représentent un groupe alkyle en C₂, et R₆ est H;
(c) R₁ est H, R₂ est H, R₃ est H, R₄ est H, deux groupes R₅ ensemble représentent un groupe alkyle en C₃, et R₆ est H;
(d) R₁ est un groupe alkyle en C₁-C₆, R₂ est un groupe alkyle en C₁-C₆, R₃ est H, R₄ est H, R₅ est H, et R₆ est H;
(e) R₁ est un groupe oxyalkyle en C₁-C₆, R₂ est un groupe oxyalkyle en C₁-C₆, R₃ est H, R₄ est H, deux groupes R₅ ensemble représentent un groupe alkyle en C₂, et R₆ est H;
(f) R₁ est H, R₂ est un groupe oxyalkyle en C₁-C₆, R₃ est H, R₄ est H, deux groupes R₅ ensemble représentent un groupe alkyle en C₂, et R₆ est H;
(g) R₁ est H, R₂ est un groupe tolyloxyalkyle en C₁-C₆, R₃ est H, R₄ est H, deux groupes R₅ ensemble représentent un groupe alkyle en C₂, et R₆ est H;
(h) R₁ est H, R₂ est H, R₃ est H, R₄ est H, deux groupes R₅ ensemble représentent un groupe alkyle en C₂, et R₆ est un groupe hydroxyalkyle en C₁-C₆;
(i) R₁ est H, R₂ est un groupe tolyloxyalkyle en C₁-C₆, R₃ est H, R₄ est H, deux groupes R₅ ensemble représentent un groupe alkyle en C₃, et R₆ est H;
(j) R₁ est H, R₂ est H, R₃ est H, R₄ est H, R₅ est H, et R₆ est un groupe aminoalkyle en C₁-C₆;
(k) R₁ est H, R₂ est H, R₃ est H, R₄ est H, deux groupes R₅ ensemble représentent un groupe alkyle en C₄, et R₆ est H;
(l) R₁ est H, R₂ est H, R₃ est H, R₄ est H, R₅ est H, et R₆ est un groupe alkyle en C₁-C₆;
(m) R₁ est H, R₂ est H, R₃ est H, R₄ est H, R₅ est H, et R₆ est un groupe (alkyl en C₁-C₆)amino;
(n) R₁ est H, R₂ est H, R₃ est H, R₄ est H, R₅ est H, et R₆ est un groupe hydroxyalkyle en C₁-C₆;
(o) R₁ est H, R₂ est H, R₃ est H, R₄ est H, R₅ est H, et R₆ est un groupe (alkyl en C₁-C₆)aminoalkyle en C₁-C₆;
(p) R₁ est H, R₂ est un groupe alkyle en C₁-C₆, R₃ est H, R₄ est H, R₅ est H, et R₆ est un groupe alkyle en C₁-C₆; et
(q) R₁ est H, R₂ est H, R₃ est H, R₄ est H, deux groupes R₅ ensemble représentent un groupe alkyle en C₂, et R₆ est un groupe alkyle en C₁-C₆.

5. Utilisation selon la revendication 1, dans laquelle ledit composé de formule (I) est choisi dans le groupe constitué des suivants
2,5-bis(4-amidinophényl)furane,
2,5-bis(4-amidinophényl)-3,4-diméthylfurane,
2,5-di-p-[2-(3,4,5,6-tétrahydropyrimidyl)phényl]furane,
2,5-bis[4-(2-imidazolinyl)phényl]furane,
2,5-[bis{4-(2-tétrahydropyrimidinyl)}phényl]-3-p-(tolyloxy)furane,
2,5-[bis{4-(2-imidazolinyl)}phényl]-3-p-(tolyloxy)furane,
2,5-bis{4[2-(N-2-hydroxyéthyl)imidazolinyl]phényl}furane,
2,5-bis[4-(N-isopropylamidino)phényl]furane,
2,5-bis{4-[3-(diméthylaminopropyl)amidino]phényl} furane,
2,5-bis{4-[N-(3-aminopropyl)amidino]phényl}furane,
2,5-bis[2-(imidazolinyl)phényl]-3,4-bis(méthoxyméthyl}furane,
2,5-bis[4-N-(diméthylaminoéthyl)amidino]phénylfurane,
2,5-bis{4-[(N-2-hydroxyéthyl)amidino]phényl}furane,
2,5-bis[4-N-(cyclopropylamidino)phényl]furane,
2,5-bis[4-(N,N-diéthylaminopropyl)amidino]phénylfurane,
2,5-bis{4-[2-(N-éthylimidazolinyl)]phényl}furane,
2,5-bis{4-[N-(3-pentylamidino)amidino]phénylfurane,
2,5-bis[4-(2-imidazolinyl)phényl]-3-méthoxyfurane,
2,5-bis[4-(N-isopropylamidino)phényl]-3-méthylfurane,
et de leurs sels pharmaceutiquement acceptables.

6. Utilisation d'un composé de formule (I) telle que définie dans la revendication 1 dans la fabrication d'un médicament destiné au traitement de *Giardia lamblia* chez un sujet ayant besoin d'un tel traitement, par administration audit sujet d'une quantité combattant *Giardia lamblia* du composé de formule (I).

7. Utilisation selon la revendication 7, dans laquelle ledit sujet est touché par *Giardia lamblia*.

8. Utilisation selon la revendication 7, dans laquelle ledit sujet est susceptible de développer *Giardia lamblia* et ledit composé est administré en une quantité prophylactiquement efficace.

9. Utilisation selon la revendication 6, dans laquelle ledit composé de formule (I) est choisi dans le groupe constitué des composés dans lesquels
(a) chaque R₁ est H, R₂ est H, R₃ est H, R₄ est H, R₅ est H, et R₆ est H;
(b) R₁ est H, R₂ est H, R₃ est H, R₄ est H, deux groupes R₅ ensemble représentent un groupe alkyle en C₂, et R₆ est H;
(c) R₁ est H, R₂ est H, R₃ est H, R₄ est H, deux groupes R₅ ensemble représentent un groupe alkyle en C₃, et R₆ est H;
(d) R₁ est un groupe alkyle en C₁-C₆, R₂ est un groupe alkyle en C₁-C₆, R₃ est H, R₄ est H, R₅ est H, et R₆ est H;
(e) R₁ est un groupe oxyalkyle en C₁-C₆, R₂ est un groupe oxyalkyle en C₁-C₆, R₃ est H, R₄ est H, deux groupes R₅ ensemble représentent un groupe alkyle en C₂, et R₆ est H;
(f) R₁ est H, R₂ est un groupe oxyalkyle en C₁-C₆, R₃ est H, R₄ est H, deux groupes R₅ ensemble représentent un groupe alkyle en C₂, et R₆ est H;
(g) R₁ est H, R₂ est un groupe tolyloxyalkyle en C₁-C₆, R₃ est H, R₄ est H, deux groupes R₅ ensemble représentent un groupe alkyle en C₂, et R₆ est H;
(h) R₁ est H, R₂ est H, R₃ est H, R₄ est H, deux groupes R₅ ensemble représentent un groupe alkyle en C₂, et R₆ est un groupe hydroxyalkyle en C₁-C₆;
(i) R₁ est H, R₂ est un groupe tolyloxyalkyle en C₁-C₆, R₃ est H, R₄ est H, deux groupes R₅ ensemble représentent un groupe alkyle en C₃, et R₆ est H;
(j) R₁ est H, R₂ est H, R₃ est H, R₄ est H, R₅ est H, et R₆ est un groupe aminoalkyle en C₁-C₆;
(k) R₁ est H, R₂ est H, R₃ est H, R₄ est H, deux groupes R₅ ensemble représentent un groupe alkyle en C₄, et R₆ est H;
(l) R₁ est H, R₂ est H, R₃ est H, R₄ est H, R₅ est H, et R₆ est un groupe alkyle en C₁-C₆;
(m) R₁ est H, R₂ est H, R₃ est H, R₄ est H, R₅ est H, et R₆ est un groupe (alkyl en C₁-C₆)amino;
(n) R₁ est H, R₂ est H, R₃ est H, R₄ est H, R₅ est H, et R₆ est un groupe hydroxyalkyle en C₁-C₆;
(o) R₁ est H, R₂ est H, R₃ est H, R₄ est H, R₅ est H, et R₆ est un groupe (alkyl en C₁-C₆)aminoalkyle en C₁-C₆;
(p) R₁ est H, R₂ est un groupe alkyle en C₁-C₆, R₃ est H, R₄ est H, R₅ est H, et R₆ est un groupe alkyle en C₁-C₆; et
(q) R₁ est H, R₂ est H, R₃ est H, R₄ est H, deux groupes R₅ ensemble représentent un groupe alkyle en C₂, et R₆ est un groupe alkyle en C₁-C₆.

10. Utilisation selon la revendication 6, dans laquelle ledit composé de formule (I) est choisi dans le groupe constitué des suivants
2,5-bis(4-amidinophényl)furane,
2,5-bis(4-amidinophényl)-3,4-diméthylfurane,
2,5-di-p-[2-(3,4,5,6-tétrahydropyrimidyl)phényl]furane,
2,5-bis[4-(2-imidazolinyl)phényl]furane,
2,5-[bis{4-(2-tétrahydropyrimidinyl) }phényl]-3-p-(tolyloxy)furane,
2,5-[bis{4-(2-imidazolinyl)}phényl]-3-p-(tolyloxy)furane,
2,5-bis {4[2-(N-2-hydroxyéthyl)imidazolinyl]phényl}furane,
2,5-bis[4-(N-isopropylamidino)phényl]furane,
2,5-bis {4-[3-(diméthylaminopropyl)amidino]phényl}furane,
2,5-bis {4-[N-(3-aminopropyl)amidino]phényl}furane,
2,5-bis[2-(imidazolinyl)phényl]-3,4-bis(méthoxyméthyl}furane,
2,5-bis[4-N-(diméthylaminoéthyl)amidino]phénylfurane,
2,5-bis{4-[(N-2-hydroxyéthyl)amidino]phényl}furane,
2,5-bis[4-N-(cyclopropylamidino)phényl]furane,
2,5-bis[4-(N,N-diéthylaminopropyl)amidino]phénylfurane,
2,5-bis {4-[2-(N-éthylimidazolinyl)]phényl}furane,
2,5-bis {4-[N-(3-pentylamidino)amidino]phénylfurane,
2,5-bis[4-(2-imidazolinyl)phényl]-3-méthoxyfurane,
2,5-bis[4-(N-isopropylamidino)phényl]-3-méthylfurane,
et de leurs sels pharmaceutiquement acceptables.

11. Utilisation d'un composé de formule (I) telle que définie dans la revendication 1 ci-dessus dans la fabrication d'un médicament destiné au traitement de *Cryptosporidium parvum* chez un sujet ayant besoin d'un tel traitement, par administration audit sujet d'une quantité combattant *Cryptosporidium parvum* du composé de formule (I).

12. Utilisation selon la revendication 11, dans laquelle ledit sujet est touché par *Cryptosporidium parvum.*

13. Utilisation selon la revendication 11, dans laquelle ledit sujet est susceptible de développer *Cryptosporidium parvum* et ledit composé est administré en une quantité prophylactiquement efficace.

14. Utilisation selon la revendication 11, dans laquelle ledit composé de formule (I) est choisi dans le groupe constitué des composés dans lesquels
(a) chaque R₁ est H, R₂ est H, R₃ est H, R₄ est H, R₅ est H, et R₆ est H;
(b) R₁ est H, R₂ est H, R₃ est H, R₄ est H, deux groupes R₅ ensemble représentent un groupe alkyle en C₂, et R₆ est H;
(c) R₁ est H, R₂ est H, R₃ est H, R₄ est H, deux groupes R₅ ensemble représentent un groupe alkyle en C₃, et R₆ est H;
(d) R₁ est un groupe alkyle en C₁-C₆, R₂ est un groupe alkyle en C₁-C₆, R₃ est H, R₄ est H, R₅ est H, et R₆ est H;
(e) R₁ est un groupe oxyalkyle en C₁-C₆, R₂ est un groupe oxyalkyle en C₁-C₆, R₃ est H, R₄ est H, deux groupes R₅ ensemble représentent un groupe alkyle en C₂, et R₆ est H;
(f) R₁ est H, R₂ est un groupe oxyalkyle en C₁-C₆, R₃ est H, R₄ est H, deux groupes R₅ ensemble représentent un groupe alkyle en C₂, et R₆ est H;
(g) R₁ est H, R₂ est un groupe tolyloxyalkyle en C₁-C₆, R₃ est H, R₄ est H, deux groupes R₅ ensemble représentent un groupe alkyle en C₂, et R₆ est H;
(h) R₁ est H, R₂ est H, R₃ est H, R₄ est H, deux groupes R₅ ensemble représentent un groupe alkyle en C₂, et R₆ est un groupe hydroxyalkyle en C₁-C₆;
(i) R₁ est H, R₂ est un groupe tolyloxyalkyle en C₁-C₆, R₃ est H, R₄ est H, deux groupes R₅ ensemble représentent un groupe alkyle en C₃, et R₆ est H;
(j) R₁ est H, R₂ est H, R₃ est H, R₄ est H, R₅ est H, et R₆ est un groupe aminoalkyle en C₁-C₆;
(k) R₁ est H, R₂ est H, R₃ est H, R₄ est H, deux groupes R₅ ensemble représentent un groupe alkyle en C₄, et R₆ est H; (l) R₁ est H, R₂ est H, R₃ est H, R₄ est H, R₅ est H, et R₆ est un groupe alkyle en C₁-C₆;
(m) R₁ est H, R₂ est H, R₃ est H, R₄ est H, R₅ est H, et R₆ est un groupe (alkyl en C₁-C₆)amino;
(n) R₁ est H, R₂ est H, R₃ est H, R₄ est H, R₅ est H, et R₆ est un groupe hydroxyalkyle en C₁-C₆;
(o) R₁ est H, R₂ est H, R₃ est H, R₄ est H, R₅ est H, et R₆ est un groupe (alkyl en C₁-C₆)aminoalkyle en C₁-C₆;
(p) R₁ est H, R₂ est un groupe alkyle en C₁-C₆, R₃ est H, R₄ est H, R₅ est H, et R₆ est un groupe alkyle en C₁-C₆; et
(q) R₁ est H, R₂ est H, R₃ est H, R₄ est H, deux groupes R₅ ensemble représentent un groupe alkyle en C₂, et R₆ est un groupe alkyle en C₁-C₆.

15. Utilisation selon la revendication 11, dans laquelle ledit composé de formule (I) est choisi dans le groupe constitué des suivants
2,5-bis(4-amidinophényl)furane,
2,5-bis(4-amidinophényl)-3,4-diméthylfurane,
2,5-di-p-[2-(3,4,5,6-tétrahydropyrimidyl)phényl]furane,
2,5-bis[4-(2-imidazolinyl)phényl]furane,
2,5-[bis{4-(2-tétrahydropyrimidinyl)}phényl]-3-p-(tolyloxy)furane,
2,5-[bis{4-(2-imidazolinyl)}phényl]-3-p-(tolyloxy)furane,
2,5-bis {4[2-(N-2-hydroxyéthyl)imidazolinyl]phényl}furane,
2,5-bis[4-(N-isopropylamidino)phényl]furane,
2,5-bis{4-[3-(diméthylaminopropyl)amidino]phényl }furane,
2,5-bis{4-[N-(3-aminopropyl)amidino]phényl }furane,
2,5-bis[2-(imidazolinyl)phényl]-3,4-bis(méthoxyméthyl}furane,
2,5-bis[4-N-(diméthylaminoéthyl)amidino]phénylfurane,
2,5-bis{4-[(N-2-hydroxyéthyl)amidino]phényl}furane,
2,5-bis[4-N-(cyclopropylamidino)phényl]furane,
2,5-bis[4-(N,N-diéthylaminopropyl)amidino]phénylfurane,
2,5-bis{4-[2-(N-éthylimidazolinyl)]phényl}furane,
2,5-bis{4-[N-(3-pentylamidino)amidino]phénylfurane,
2,5-bis[4-(2-imidazolinyl)phényl]-3-méthoxyfurane,
2,5-bis[4-(N-isopropylamidino)phényl]-3-méthylfurane,
et de leurs sels pharmaceutiquement acceptables.

16. Composé de formule (I): 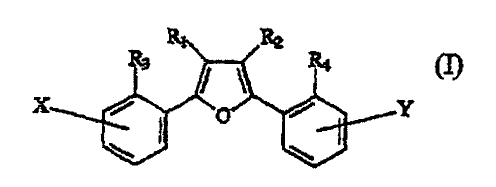 dans laquelle:
X et Y sont situés en position *para* et sont chacun 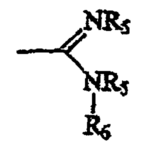 et dans laquelle lesdits composés de formule (I) sont choisis dans le groupe constitué des composés dans lesquels:
(a) R₁ est H, R₂ est H ou un groupe alkyle en C₁-C₆, R₃ est H, R₄ est H, R₅ est H, et R₆ est un groupe isoalkyle;
(b) R₁ est H, R₂ est H, R₃ est H, R₄ est H, R₅ est H, et R₆ est un groupe (alcoxy en C₃-C₈)alkyle;
(c) R₁ est H, R₂ est H ou un groupe alkyle en C₁-C₆, R₃ est H, R₄ est H, R₅ est H, et R₆ est un groupe éthylhydroxy, propylhydroxy, butylhydroxy, pentylhydroxy, ou hexylhydroxy;
(d) R₁ est H, R₂ est H ou un groupe alkyle en C₁-C₆, R₃ est H, R₄ est H, R₅ est H, et R₆ est un groupe propoxyéthyle;
(e) R₁ est H, R₂ est H ou un groupe alkyle en C₁-C₆, R₃ est H, R₄ est H, R₅ est H, et R₆ est un groupe propoxyisopropyle;
(f) R₁ est H, R₂ est H ou un groupe alkyle en C₁-C₆, R₃ est H, R₄ est H, R₅ est H, et R₆ est un groupe tolyloxy, benzyle, tolyloxyalkyle en C₁-C₆ ou benzylalkyle en C₁-C₆; et
(g) R₁ est H, R₂ est H ou un groupe alkyle en C₁-C₆, R₃ est H, R₄ est H, R₅ est H, et R₆ est un groupe (alkyl en C₁-C₆) cycloalkyle en C₁-C₆;
et de leurs sels pharmaceutiquement acceptables.
